# EUROPEAN PATENT SPECIFICATION

(11) **EP 3 004 893 B1**
(45) Date of publication and mention of the grant of the patent: **16.03.2022**
(21) Application number: 14733678.8
(22) Date of filing: 06.06.2014
(51) Int. Cl.: G01N 33/68

(54) **METHODS AND COMPOSITIONS RELATING TO NEURODEGENERATIVE DISEASES**
VERFAHREN UND ZUSAMMENSETZUNGEN IN ZUSAMMENHANG MIT NEURODEGENERATIVEN ERKRANKUNGEN
MÉTHODES ET COMPOSITIONS SE RAPPORTANT À DES MALADIES NEURODÉGÉNÉRATIVES

(30) Priority: 07.06.2013 GB 201310150
(43) Date of publication of application: 13.04.2016
(73) Proprietor: Electrophoretics Limited, London WC1H 9BB (GB)
(72) Inventor: WARD, Malcolm Andrew, Cobham Surrey KT11 3EP (GB); LIANG, Hui-Chung, Cobham Surrey KT11 3EP (GB); PIKE, Ian Hugo, Cobham Surrey KT11 3EP (GB)
(74) Representative: Greenwood, Matthew David
(86) International application number: PCT/GB2014/051758
(87) International publication number: WO 2014/195728

(56) References cited:
- US-A1- 2008 289 964
- CARINA SIHLBOM ET AL: "Structural and Quantitative Comparison of Cerebrospinal Fluid Glycoproteins in Alzheimerâ s Disease Patients and Healthy Individuals", NEUROCHEMICAL RESEARCH, KLUWER ACADEMIC PUBLISHERS-PLENUM PUBLISHERS, NE, vol. 33, no. 7, 21 February 2008 (2008-02-21), pages 1332-1340, XP019612525, ISSN: 1573-6903
- BARNARD G ET AL: "THE MEASUREMENT OF PRION PROTEIN IN BOVINE BRAIN TISSUE USING DIFFERENTIAL EXTRACTION AND DELFIA AS A DIAGNOSTIC TEST FOR BSE", LUMINESCENCE: THE JOURNAL OF BIOLOGICAL AND CHEMICAL LUMINESCENCE; [31660], JOHN WILEY & SONS LTD, GB, vol. 15, no. 6, 1 November 2000 (2000-11-01), pages 357-362, XP009036948, ISSN: 1522-7235, DOI: 10.1002/1522-7243(200011/12)15:6<357::AID- BIO621>3.0.CO;2-V
- Maja Amedjkouh Puchades: "Development of proteomic methods for studying cerebrospinal fluid proteins involved in Alzheimer 's disease.", , 1 January 2003 (2003-01-01), pages 1-68, XP055139821, Retrieved from the Internet: URL:http://hdl.handle.net/2077/15791 [retrieved on 2014-09-12]
- JAMES T. KAPRON ET AL: "Identification and characterization of glycosylation sites in human serum clusterin", PROTEIN SCIENCE, vol. 6, no. 10, 1 October 1997 (1997-10-01), pages 2120-2133, XP055139782, ISSN: 0961-8368, DOI: 10.1002/pro.5560061007
- LAMOUREUX LISE ET AL: "Analysis of clusterin glycoforms in the urine of BSE-infected Fleckvieh-Simmental cows", JOURNAL OF TOXICOLOGY AND ENVIRONMENTAL HEALTH. PART A, TAYLOR & FRANCIS, US, vol. 74, no. 2-4, 1 January 2011 (2011-01-01), pages 138-145, XP009180131, ISSN: 1528-7394, DOI: 10.1080/15287394.2011.529063 [retrieved on 2011-01-06]

### SEQUENCE LISTING

<110> Electrophoretics Limited
<120> Methods and Compositions Relating to Neurodegenerative Diseases
<130> CSC/BP6981856
<150> GB 1310150.6
   <151> 2013-06-07
<160> 11
<170> PatentIn version 3.3
<210> 1
   <211> 449
   <212> PRT
   <213> Homo sapiens
<400> 1
   Met Met Lys Thr Leu Leu Leu Phe Val Gly Leu Leu Leu Thr Trp Glu
   1               5                   10                  15
   Ser Gly Gln Val Leu Gly Asp Gln Thr Val Ser Asp Asn Glu Leu Gln
               20                  25                  30
   Glu Met Ser Asn Gln Gly Ser Lys Tyr Val Asn Lys Glu Ile Gln Asn
           35                  40                  45
   Ala Val Asn Gly Val Lys Gln Ile Lys Thr Leu Ile Glu Lys Thr Asn
       50                  55                  60
   Glu Glu Arg Lys Thr Leu Leu Ser Asn Leu Glu Glu Ala Lys Lys Lys
   65                  70                  75                  80
   Lys Glu Asp Ala Leu Asn Glu Thr Arg Glu Ser Glu Thr Lys Leu Lys
                   85                  90                  95
   Glu Leu Pro Gly Val Cys Asn Glu Thr Met Met Ala Leu Trp Glu Glu
               100                 105                 110
   Cys Lys Pro Cys Leu Lys Gln Thr Cys Met Lys Phe Tyr Ala Arg Val
           115                 120                 125
   Cys Arg Ser Gly Ser Gly Leu Val Gly Arg Gln Leu Glu Glu Phe Leu
       130                 135                 140
   Asn Gln Ser Ser Pro Phe Tyr Phe Trp Met Asn Gly Asp Arg Ile Asp
   145                 150                 155                 160
   Ser Leu Leu Glu Asn Asp Arg Gln Gln Thr His Met Leu Asp Val Met
   165 170 175
   Gln Asp His Phe Ser Arg Ala Ser Ser Ile Ile Asp Glu Leu Phe Gln
   180 185 190
   Asp Arg Phe Phe Thr Arg Glu Pro Gln Asp Thr Tyr His Tyr Leu Pro
   195 200 205
   Phe Ser Leu Pro His Arg Arg Pro His Phe Phe Phe Pro Lys Ser Arg
   210 215 220
   Ile Val Arg Ser Leu Met Pro Phe Ser Pro Tyr Glu Pro Leu Asn Phe
   225 230 235 240
   His Ala Met Phe Gln Pro Phe Leu Glu Met Ile His Glu Ala Gln Gln
   245 250 255
   Ala Met Asp Ile His Phe His Ser Pro Ala Phe Gln His Pro Pro Thr
   260 265 270
   Glu Phe Ile Arg Glu Gly Asp Asp Asp Arg Thr Val Cys Arg Glu Ile
   275 280 285
   Arg His Asn Ser Thr Gly Cys Leu Arg Met Lys Asp Gln Cys Asp Lys
   290 295 300
   Cys Arg Glu Ile Leu Ser Val Asp Cys Ser Thr Asn Asn Pro Ser Gln
   305 310 315 320
   Ala Lys Leu Arg Arg Glu Leu Asp Glu Ser Leu Gln Val Ala Glu Arg
   325 330 335
   Leu Thr Arg Lys Tyr Asn Glu Leu Leu Lys Ser Tyr Gln Trp Lys Met
   340 345 350
   Leu Asn Thr Ser Ser Leu Leu Glu Gln Leu Asn Glu Gln Phe Asn Trp
   355 360 365
   Val Ser Arg Leu Ala Asn Leu Thr Gln Gly Glu Asp Gln Tyr Tyr Leu
   370 375 380
   Arg Val Thr Thr Val Ala Ser His Thr Ser Asp Ser Asp Val Pro Ser
   385 390 395 400
   Gly Val Thr Glu Val Val Val Lys Leu Phe Asp Ser Asp Pro Ile Thr
   405 410 415
   Val Thr Val Pro Val Glu Val Ser Arg Lys Asn Pro Lys Phe Met Glu
   420 425 430
   Thr Val Ala Glu Lys Ala Leu Gln Glu Tyr Arg Lys Lys His Arg Glu
   435 440 445
   Glu
<210> 2
   <211> 8
   <212> PRT
   <213> Homo sapiens
<220>
   <221> SITE
   <222> (2)..(2)
   <223> Glycan attachment site
<400> 2
   His Asn Ser Thr Gly Cys Leu Arg
   1 5
<210> 3
   <211> 9
   <212> PRT
   <213> Homo sapiens
<220>
   <221> SITE
   <222> (6)..(6)
   <223> Glycan attachment site
<400> 3
   Lys Glu Asp Ala Leu Asn Glu Thr Arg
   1 5
<210> 4
   <211> 10
   <212> PRT
   <213> Homo sapiens
<220>
   <221> SITE
   <222> (7)..(7)
   <223> Glycan attachment site
<400> 4
   Lys Lys Glu Asp Ala Leu Asn Glu Thr Arg
   1 5 10
<210> 5
   <211> 11
   <212> PRT
   <213> Homo sapiens
<220>
   <221> SITE
   <222> (8)..(8)
   <223> Glycan attachment site
<400> 5
   Lys Lys Lys Glu Asp Ala Leu Asn Glu Thr Arg
   1               5                   10
<210> 6
   <211> 20
   <212> PRT
   <213> Homo sapiens
<220>
   <221> SITE
   <222> (3)..(3)
   <223> Glycan attachment site
<400> 6 Trp Val Ser Arg 20
<210> 7
   <211> 14
   <212> PRT
   <213> Homo sapiens
<220>
   <221> SITE
   <222> (3)..(3)
   <223> Glycan attachment site
<400> 7
   Leu Ala Asn Leu Thr Gln Gly Glu Asp Gln Tyr Tyr Leu Arg
   1               5                   10
<210> 8
   <211> 20
   <212> PRT
   <213> Homo sapiens
<220>
   <221> SITE
   <222> (7)..(7)
   <223> Glycan attachment site
<400> 8
   Gln Leu Glu Glu Phe Leu Asn Gln Ser Ser Pro Phe Tyr Phe Trp Met
   1               5                   10                  15
   Trp Gly Asp Arg
               20
<210> 9
   <211> 18
   <212> PRT
   <213> Homo sapiens
<220>
   <221> SITE
   <222> (7)..(7)
   <223> Glycan attachment site
<400> 9
   Glu Leu Pro Gly Val Cys Asn Glu Thr Met Met Ala Leu Trp Glu Glu
   1               5                   10                  15
   Cys Lys
<210> 10
   <211> 24
   <212> PRT
   <213> Homo sapiens
<220>
   <221> SITE
   <222> (9)..(9)
   <223> Glycan attachment site
<400> 10
   Leu Lys Glu Leu Pro Gly Val Cys Asn Glu Thr Met Met Ala Leu Trp
   1               5                   10                  15
   Glu Glu Cys Lys Pro Cys Leu Lys
               20
<210> 11
   <211> 8
   <212> PRT
   <213> Homo sapiens
<220>
   <221> SITE
   <222> (5)..(5)
   <223> Glycan attachment site
<400> 11
   Glu Asp Ala Leu Asn Glu Thr Arg
   1               5

## Description

### Field of the Invention

The present invention relates to methods relating to neurodegenerative diseases, including Alzheimer's disease. Specifically, the present invention identifies and describes protein isoforms that are differentially expressed in the Alzheimer's disease state relative to their expression in the normal state and, in particular, identifies and describes proteins associated with Alzheimer's disease.

### Background of the Invention

Dementia is one of the major public health problems of the elderly, and in our ageing populations the increasing numbers of patients with dementia is imposing a major financial burden on health systems around the world. More than half of the patients with dementia have Alzheimer's disease (AD). The prevalence and incidence of AD have been shown to increase exponentially. The prevalence for AD in Europe is 0.3% for ages 60-69 years, 3.2% for ages 70-79 years, and 10.8% for ages 80-89 years (Rocca, Hofman et al. 1991). The survival time after the onset of AD is approximately from 5 to 12 years (Friedland 1993).

Alzheimer's disease (AD), the most common cause of dementia in older individuals, is a debilitating neurodegenerative disease for which there is currently no cure. It destroys neurons in parts of the brain, chiefly the hippocampus, which is a region involved in coding memories. Alzheimer's disease gives rise to an irreversible progressive loss of cognitive functions and of functional autonomy. The earliest signs of AD may be mistaken for simple forgetfulness, but in those who are eventually diagnosed with the disease, these initial signs inexorably progress to more severe symptoms of mental deterioration. While the time it takes for AD to develop will vary from person to person, advanced signs include severe memory impairment, confusion, language disturbances, personality and behaviour changes, and impaired judgement. Persons with AD may become non-communicative and hostile. As the disease ends its course in profound dementia, patients are unable to care for themselves and often require institutionalisation or professional care in the home setting. While some patients may live for years after being diagnosed with AD, the average life expectancy after diagnosis is eight years.

In the past, AD could only be definitively diagnosed by brain biopsy or upon autopsy after a patient died. These methods, which demonstrate the presence of the characteristic plaque and tangle lesions in the brain, are still considered the gold standard for the pathological diagnoses of AD. However, in the clinical setting brain biopsy is rarely performed and diagnosis depends on a battery of neurological, psychometric and biochemical tests, including the measurement of biochemical markers such as the ApoE and tau proteins or the beta-amyloid peptide in cerebrospinal fluid and blood.

Biomarkers may possibly possess the key in the next step for diagnosing AD and other dementias. A biological marker that fulfils the requirements for the diagnostic test for AD would have several advantages. An ideal biological marker would be one that identifies AD cases at a very early stage of the disease, before there is degeneration observed in the brain imaging and neuropathological tests. A biomarker could be the first indicator for starting treatment as early as possible, and also very valuable in screening the effectiveness of new therapies, particularly those that are focussed on preventing the development of neuropathological changes. Repetitive measurement of the biological markers of the invention would also be useful in following the development and progression of the disease.

Markers related to pathological characteristics of AD; plaques and tangles (Aβ and tau) have been the most extensively studied. The most promising has been from studies of CSF concentration of Aβ(1-40), Aβ(1-42) and tau or the combination of both proteins in AD. Many studies have reported a decrease in Aβ(1-42) in CSF, while the total Aβ protein or Aβ(1-40) concentration remain unchanged (Ida, Hartmann et al. 1996; Kanai, Matsubara et al. 1998; Andreasen, Hesse et al. 1999).

Recognising that CSF is a less desirable sample and that 'classical' markers of AD pathology including amyloid and tau are not reliably detectable in other fluids, there have been several efforts to identify additional protein markers in blood and blood products such as serum and plasma. One group of blood proteins that are differentially expressed in the AD state relative to their expression in the normal state are described in WO2006/035237 and includes the protein clusterin which has previously been associated with AD pathology in the brain of affected individuals. The value of clusterin as a potential biomarker in AD has been explored by various groups in both cerebrospinal fluid (CSF) and blood, often with contradictory results. One possible explanation for the discrepancy between CSF clusterin levels and those found in the brain is the effect of protein glycosylation which may serve to mask epitopes recognised by antibodies used in immunoassays to measure clusterin. Indeed, Nilselid et al. (2006) demonstrated that accurate quantification of clusterin in human CSF was only possible when all glycan moieties were enzymatically removed from clusterin prior to measurement by ELISA. In their study, they found that the clusterin amount measured by two specific antibodies to the alpha and beta chains of clusterin increased by approximately 70% following deglycosylation. Importantly, although clusterin levels were generally elevated in male AD patients relative to healthy male controls their study failed to show diagnostic utility for measuring CSF levels of either the naturally glycosylated clusterin levels, or those of the *ex vivo* deglycosylated protein. Furthermore, they found no difference in clusterin levels between women with AD and the female control group. The authors conclude that there was no general difference in clusterin glycosylation levels between AD and control groups but rather contradict this by suggesting that protein microheterogeneity (glycosylation, phosphorylation etc) could be another useful target in the diagnosis or prognostic monitoring of disease.

Maja Amedjkouh Puchades: "Development of proteomic methods for studying cerebrospinal fluid proteins involved in Alzheimer 's disease.",1 January 2003 (2003-01-01), pages 1-68 reports two isoforms of clusterin present in CSF that are altered in AD patients vs normal controls.

### Summary of the Invention

In light of this uncertain art and wishing to develop a minimally invasive diagnostic test using blood rather than CSF, the inventors have surprisingly shown that glycosylation of clusterin in human plasma is highly heterogenous with over 40 different isoforms identified to date. Furthermore, a small subset of only 8 of the identified glycoforms is consistently regulated between patients with AD and those with Mild Cognitive Impairment. Furthermore, levels of these same glycoforms can predict the severity and rate of progression of AD within an individual.

The present inventors have previously determined a number of plasma biomarkers for Alzheimer's disease (see US7,897,361). However, they found that immunoassays and selected reaction monitoring experiments did not fully replicate the results they obtained for the same biomarkers using 2-dimensional gel electrophoresis (2DE). The inventors investigated whether this difference could be due to specific post-translational events which were not being replicated in the validation experiments.

The inventors surprisingly found that post-translational events created distinct isoforms of the protein, e.g. glycoforms, which were differentially expressed in different forms and stages of dementia. Accordingly, the inventors have identified more potent biomarkers for dementia and as a result can provide more sophisticated methods for the diagnosis, prognosis and monitoring of dementia such as Alzheimer's disease.

Broadly, the present invention relates to a method for assessing the hippocampal atrophy level in a test subject with Alzheimer's disease (AD).

A protein *in vivo* can be present in several different forms. These different forms may be produced by alternative splicing; by alterations between alleles, e.g. single nucleotide polymorphisms (SNPs); or may be the result of post translational events such as glycosylation (glycoforms). A glycoform is an isoform of a protein that differs only with respect to the number or type of attached glycan.

The present invention provides a method for assessing the hippocampal atrophy level in a test subject with Alzheimer's disease (AD), comprising:
(i) providing a protein-containing sample that has been obtained from the test subject;
(ii) determining the concentration, amount or degree of expression of at least one clusterin glycoform, wherein tetra-antennary glycoforms of the at least one clusterin glycoform as a proportion of the total of all glycoforms of the same glycoprotein fragment are quantified;
(iii) comparing said concentration, amount or degree determined in (ii) with a reference from a control subject that does have AD with a low level of hippocampal atrophy;
(iv) based on the level of the at least one clusterin glycoform in the test subject relative to the reference, making an assessment of the hippocampal atrophy level in the test subject,
wherein a lower relative level of tetra-antennary glycoforms in the sample from the test subject compared with the relative level of tetra-antennary glycoforms in the reference from the control subject indicates that the test subject is predicted to have a relatively higher level of hippocampal atrophy,
wherein said at least one clusterin glycoform comprises a glycosylated fragment of human clusterin consisting of HN*STGCLR (SEQ ID NO: 2), wherein "N*" indicates the glycan attachment residue, and
wherein the protein-containing sample is selected from the group consisting of: blood plasma, blood cells or serum.

Said at least one clusterin glycoform may further comprise:
a) any one of the clusterin glycopeptides set forth in Table 3A, Table 3B, Table 3C, Table 5, Table 6 and/or Table 7; and/or
b) a β64N-glycan selected from the group consisting of:
   β64N_SA1-(HexNAc-Hex)2-core; β64N_SA2-(HexNAc-Hex)2-core; β64N_SA1-(HexNAc-Hex)3-core; β64N_SA2-(HexNAc-Hex)3-core; β64N_SA1-(HexNAc-Hex)4-core; β64N_SA3-(HexNAc-Hex)3-core; β64N_SA2-(HexNAc-Hex)4-core; and β64N_SA3-(HexNAc-Hex)4-core.

The skilled person will be aware that a variety of suitable techniques exist for measuring the amount or concentration of specific glycoforms. This includes the use of non-human antibodies generated by immunisation with specific isoforms of the proteins if the present invention wherein such antibodies have the required specificity for the diagnostic isoform, particularly glycoforms. In particular, the use of synthetic peptides of Sequence ID's 2-10 with the appropriate glycan structures. Such peptides are not found in nature and must therefore be prepared ex vivo through digestion of naturally occurring clusterin or by the use of in vitro synthetic chemistry.

More specifically contemplated herein are methods that include measurement using gel electrophoresis or LC-MS/MS.

In some cases the relative amount of each glycoform is calculated by comparison to an equivalent heavy-isotope labelled reference glycoform using Selected Reaction Monitoring mass spectrometry. In particular, the heavy-isotope labelled reference glycoform may be a synthetic glycopeptide in which one or more heavy isotopes of H, C, N or O are substituted within the peptide or sugar components of said glycoform.

In some cases the heavy-isotope labelled reference glycoform is an enriched, naturally occurring glycoform that has been labelled with an isotopic mass tag wherein said isotopic mass tag with one or more heavy isotopes of H, C, N or O and wherein such mass tag is able to react with the peptide or sugar components of said glycoform.

In some cases the relative amount of each glycoform is calculated by comparison to an equivalent glycoform labelled with an isobaric mass tag as generally disclosed in European Patent 2,115,475 wherein:
(i) each sample taken from the test subject is labelled with one member of an isobaric mass tag set to create a labelled analytical sample;
(ii) a standard reference panel of enriched glycoforms is separated into between two and six aliquots and each aliquot is labelled separately with additional members of the same isobaric mass tag set as the labelled analytical sample and each independently labelled aliquot of the reference panel is mixed in a predefined ratio to create a clinically relevant concentration curve as a standard reference mixture;
(iii) an equal volume of the labelled analytical sample and the standard reference mixture are mixed together to form the MScalibrator sample; and
(iv) the MScalibrator sample prepared in step (iii) is analysed by mass spectrometry. In particular, the the isobaric mass tag set may be a Tandem Mass Tag set.

In certain cases in accordance with the present invention, the glycoforms are measured using sum scaled Selected Reaction Monitoring (SRM) mass spectrometry.

In certain cases in accordance with the present invention, the glycoforms are not labelled.

In certain cases in accordance with the present invention, the method does not comprise subjecting the sample to gel electrophoretic separation, and/or does not comprise subjecting the sample to enrichment by immunoprecipitation.

In certain cases in accordance with the present invention, the glycoforms are glycoforms and are measured by a method essentially as described in Example 6.

In some cases in accordance with the present invention the at least one glycoform may be measured by an immunological assay, such as Western blotting or ELISA.

The measurement of glycan structures on clusterin may be performed by various methods. In 2-dimensional gel electrophoresis the addition or removal of sugar groups within the glycan structure will affect both the apparent molecular mass and the iso-electric focusing point of clusterin leading to a 'train' of spots within the gel. Such trains of spots are well known to the skilled practitioner. By way of example, a plasma protein from a subject suspected of suffering from dementia is subjected to 2-dimensional gel electrophoresis. After completion of the second dimension the gel is stained with a protein or sugar-selective dye to reveal individual protein spots or glycoprotein spots respectively. Typically an image of the whole gel is captured using a CCD camera and the relative abundance of each spot calculated based on staining intensity using commercially available software such as SameSpots (Non-Linear Dynamics, UK). The train of spots comprising clusterin isoforms can be identified by comparison with a reference gel. Alternatively, spots can be cut from the gel and proteins identified using mass spectrometry. Ultimately, the relative abundance of each spot representing the different clusterin isoforms is determined.

The present invention also provides a method for stratifying a plurality of test subjects according to their stage of Alzheimer's disease (AD), comprising:
carrying out the method according to the present invention on at least one test sample from each of the human test subjects; and
based on the level of the at least one specific clusterin glycoform in each of the test subjects, stratifying the test subjects into more or less advanced stage AD; wherein the test subjects are stratified according to their predicted degree of hippocampal atrophy.

The present invention also provides a method of determining the efficacy of a treatment of Alzheimer's disease (AD)in a mammalian subject, comprising:
determining the level of one or more clusterin glycoforms by carrying out the method according to the present invention before treatment of the subject and at least one time during or following treatment of the subject,
wherein successful treatment is demonstrated by the level of the said one or more clusterin glycoforms remaining stable or reverting to more normal levels, wherein, the subject is human.

The invention will now be described in more detail, by way of example and not limitation, by reference to the accompanying drawings.

### Brief Description of the Figures

**Figure 1** Theoretical and actual clusterin glycoform distributions in 2DE. **Panel A** - Mathematical modeling of Clusterin alpha and beta chain Additional series for Tetra-antennary structures can be modeled in a similar manner (not shown). **Panel B** - 2DE spots of immune-precipitated protein. 16 distinct spots were analysed and fully sialylated N-glycans were the most abundant structure at each glycosylation site in all 16 spots. The shift in pI observed for different gel spots is most likely driven by glycosylation via alterations in number of antennae and sialic acids.
**Figure 2****.** Tabular representation of individual glycoforms of four clusterin peptides detected in 16 spots on 2DE gels (Peptide A: SEQ ID NO: 2; Peptide B: SEQ ID NO: 11; Peptide C: SEQ ID NO: 7; Peptide D: SEQ ID NO: 10).
**Figure 3****.** Vector diagram illustrating the change in 2DE coordinates associated with the removal of specific glycan units from N linked carbohydrates
**Figure 4****.** Structure of the NA3 substrate (Dextra-UK Ltd, Catalogue No: C1124) Molecular Weight = 2006.82 Da Chemical Formula: C₇₆H₁₂₇N₅O₅₆

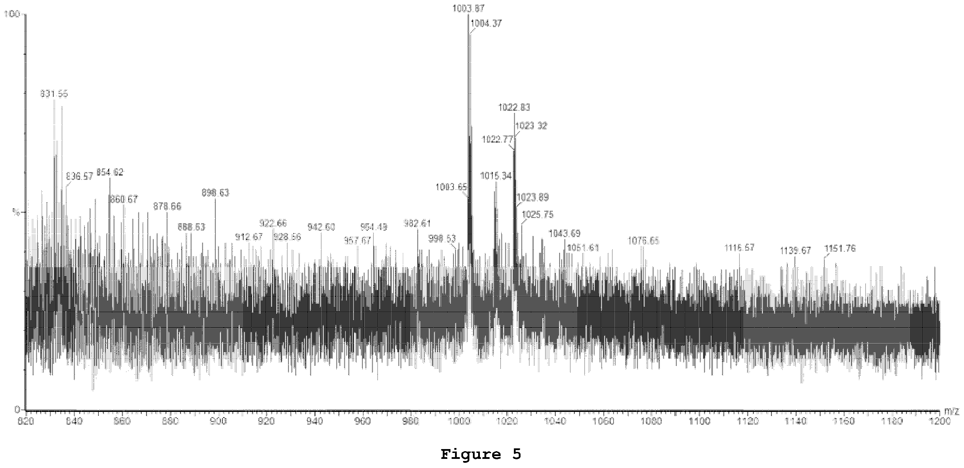

**Figure 5****.** ESI-TOF spectrum of intact NA3 substrate showing presence of doubly charged molecular ion at m/z 1003.87 and related sodium and potassium cations.
**Figure 6****.** MS/MS spectrum of m/z 1050.74 the [M+3H]³⁺ molecular ion for clusterin glycopeptide of molecular weight 3149.22 Da. The fragment ions enable the structure of the glycan to be deduced with the fragment ion at m/z 574.47 representing the [Peptide+HexNac]+ moiety. Hence the sequence of the "naked" peptide is HN*STGCLR (SEQ ID NO: 2) and a fully sialylated bi-antennary glycan structure, SA₂-(HexNAc-Hex)₂, is attached to the asparagine residue (N*)
**Figure 7****.** Mass spectrum showing molecular ions for two sialylated forms of the tetra-antennary glycopeptides HN*STGCLR (SEQ ID NO: 2) observed in instances of low atrophy and not observed in high atrophy.
**Figure 8****.** Relative percentage of tetra-antennary glycoforms within eight individuals with low and high levels of hippocampal atrophy.
**Figure 9****.** Shows box plots of significantly regulated clusterin β64N glycopeptides from Discovery Cohort (Orbitrap Fusion) A) β64N_SA1-(HexNAc-Hex)2-core; B) β64N_SA2-(HexNAc-Hex)2-core; C) β64N_SA1-(HexNAc-Hex)3-core; D) β64N_SA2-(HexNAc-Hex)3-core; E) β64N_SA3-(HexNAc-Hex)3-core; and F) βB4N_SA3-(HexNAc-Hex)4-core.
**Figure 10****.** Shows box plots of significantly regulated clusterin β64N glycopeptides from Replication Cohort (Orbitrap Fusion) A) β64N_SA1-(HexNAc-Hex)2-core; B) β64N_SA1-(HexNAc-Hex)3-core; and C) β64N_SA2-(HexNAc-Hex)3-core.
**Figure 11****.** Shows box plots of significantly regulated clusterin β64N glycopeptides from combined Discovery and Replication Cohorts (Orbitrap Fusion) A) β64N_SA1-(HexNAc-Hex)2-core; B) β64N_SA2-(HexNAc-Hex)2-core; C) β64N_SA1-(HexNAc-Hex)3-core; and D) β64N_SA2-(HexNAc-Hex)3-core.
**Figure 12****.** Shows box plots of significantly regulated clusterin β64N glycopeptides from Discovery Cohort by SRM analysis (TSQ Vantage) A) β64N_SA1-(HexNAc-Hex)2-core; B) β64N_SA2-(HexNAc-Hex)2-core; C) β64N_SA1-(HexNAc-Hex)3-core; D) β64N_SA2-(HexNAc-Hex)3-core; and E) β64N_SA1-(HexNAc-Hex)4-core.
**Figure 13****.** Shows an SDS-PAGE image of albumin/IgG-depleted normal human plasma. Red bars represent cut points and numbers represent the band number used for Orbitrap analysis to identify clusterin glycopeptides.
**Figure 14****.** Shows total ion chromatogram (TIC) of band #4 - #9 from depleted plasma using glyco-SRM method. Eight clusterin β64N glycopeptides were served as precursors (m/z 953.71, 1050.74, 1075.42, 1172.45, 1197.13, 1269.49, 1294.17, 1391.53), and fragment ions at m/z 366.14, 574.56, and 657.24 were set as transition ions for each precursor.
**Figure 15****.** Shows XIC of band #7 presenting various retention time and peak area of eight glycoforms at site β64N.

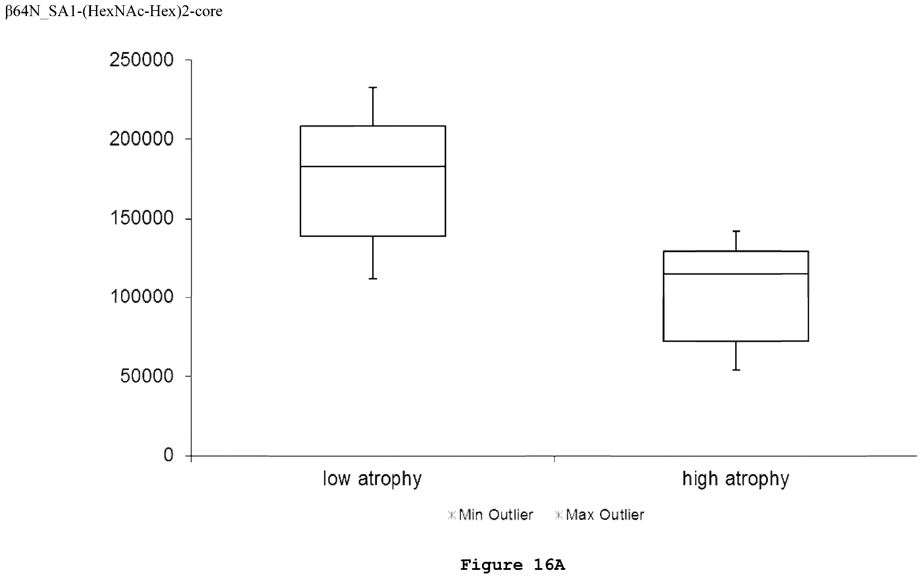

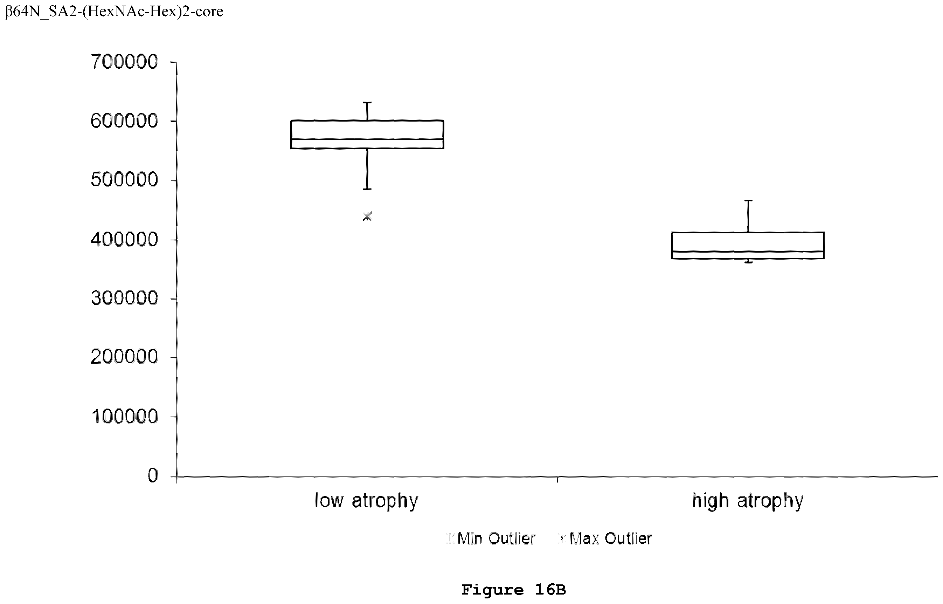

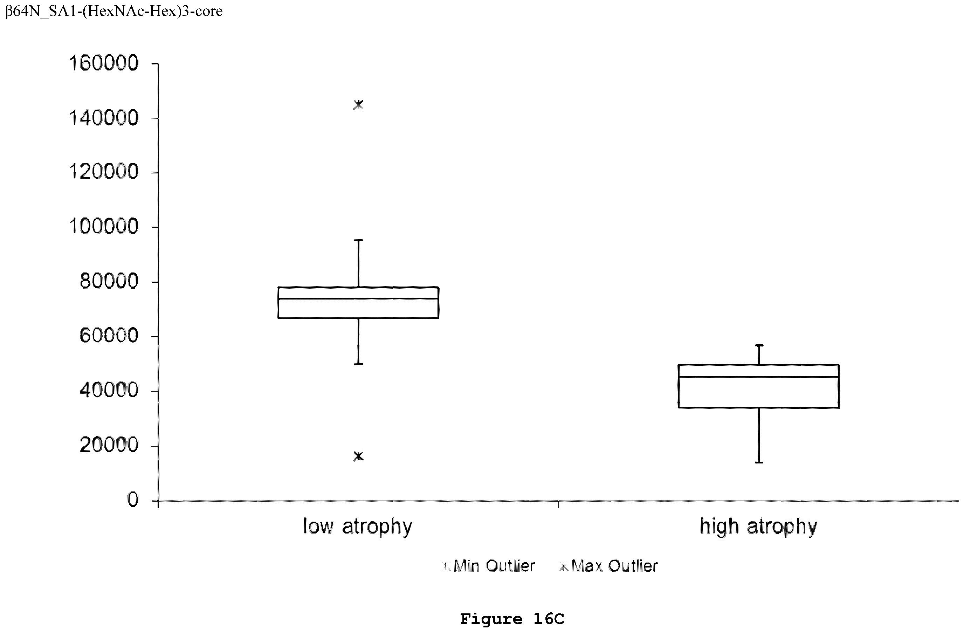

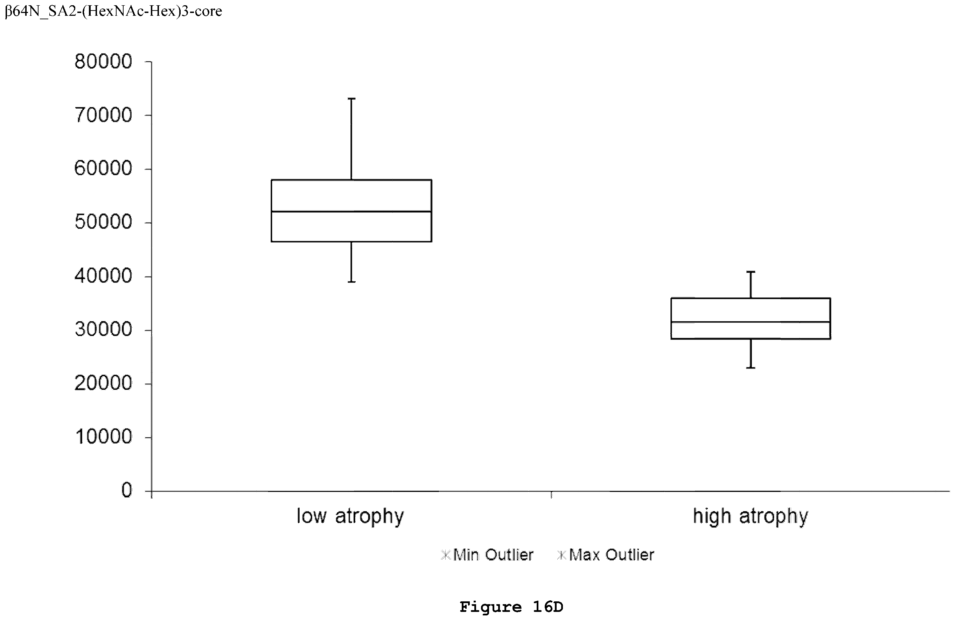

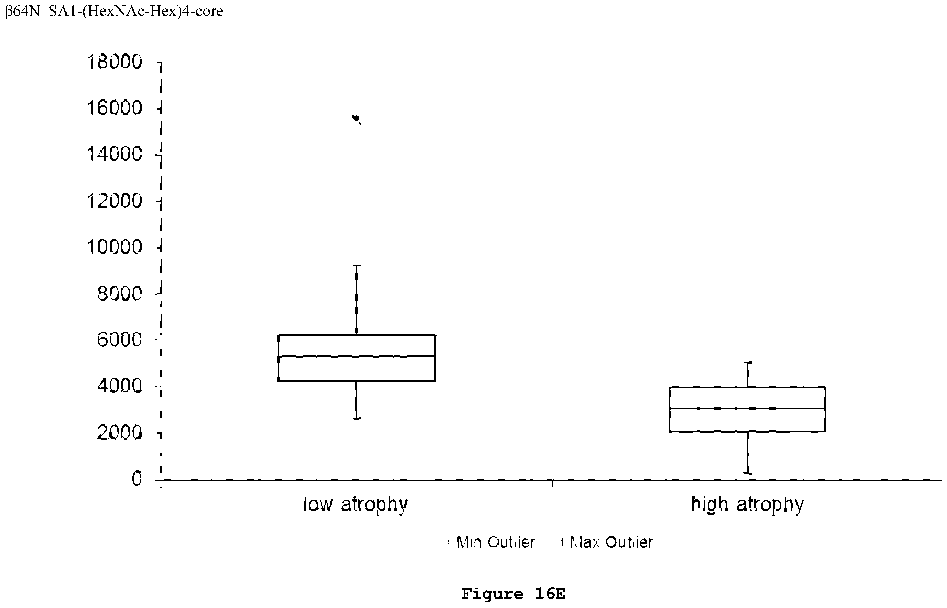

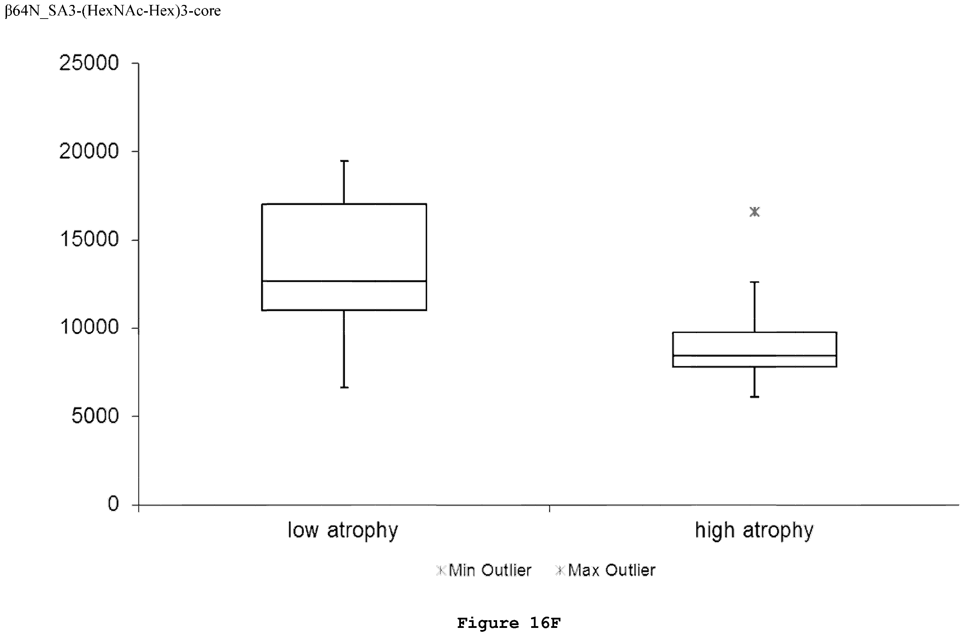

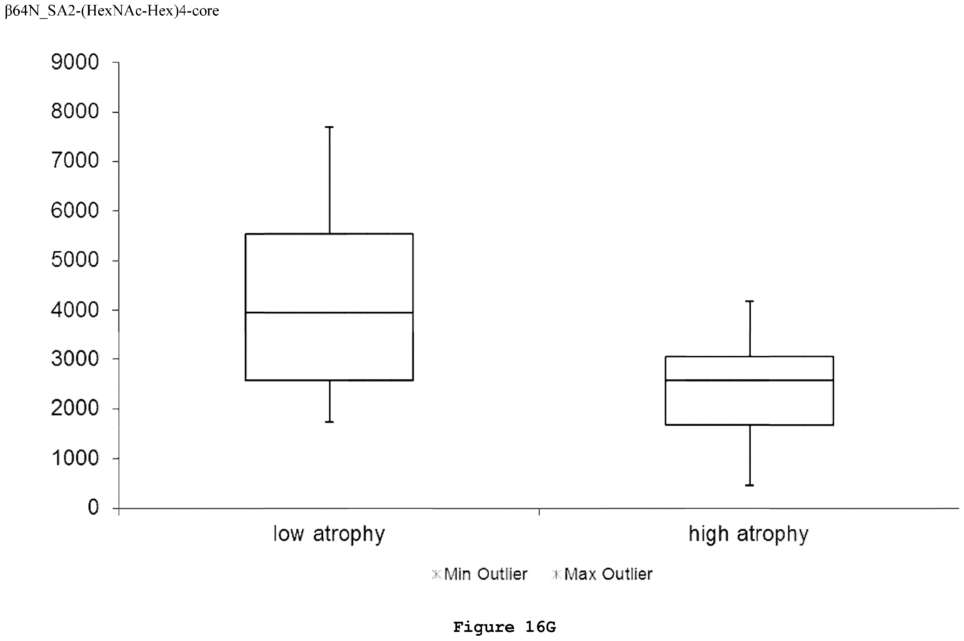

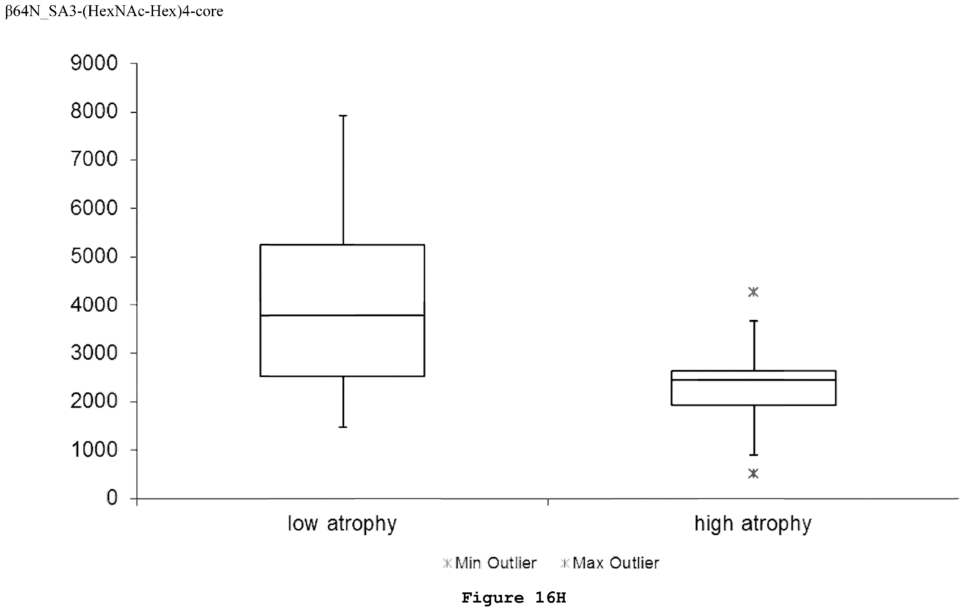

**Figure 16****.** Shows box plots of significantly regulated clusterin β64N glycopeptides from combined Discovery & Validation Cohorts by SRM analysis (TSQ Vantage) A) β64N_SA1-(HexNAc-Hex)2-core; B) β64N_SA2-(HexNAc-Hex)2-core; C) β64N_SA1-(HexNAc-Hex)3-core; D) β64N_SA2-(HexNAc-Hex)3-core; E) β64N_SA1-(HexNAc-Hex)4-core; F) β64N_SA3-(HexNAc-Hex)3-core; G) β64N_SA2-(HexNAc-Hex)4-core; and H) β64N_SA3-(HexNAc-Hex)4-core.

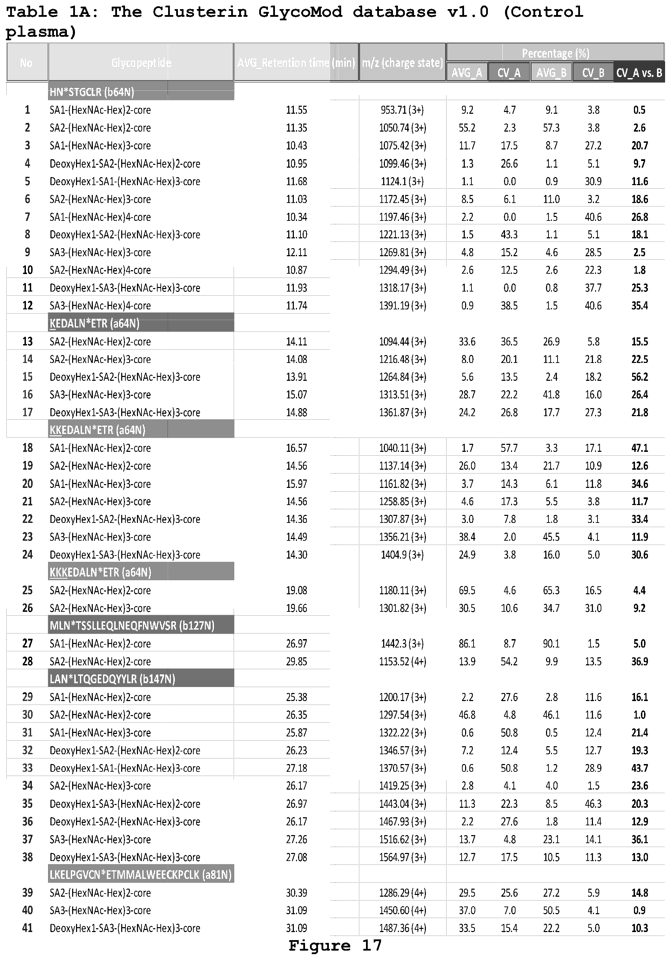

**Figure 17****.** Shows Table 1A - The Clusterin GlycoMod database v1.0 (control plasma) (SEQ ID NOs: 2 - 7 & 10, respectively).
**Figure 18****.** Shows Table 1B - The Clusterin GlycoMod database v1.1 (MCI/AD plasma) (SEQ ID NOs: 2 - 7, 9, 10, & 8 respectively).

### Detailed Description

In describing the present invention, the following terms will be employed, and are intended to be defined as indicated below.

The term "subject" includes a human or an animal. In accordance with certain embodiments of the present invention, the subject may have been previously diagnosed with AD and/or previously diagnosed with mild cognitive impairment (MCI). The subject is preferably a human. The subject may be a human of at least 60 years of age, optionally at least 70 or at least 80 years of age.

The term "diagnosis", as used herein, includes the provision of any information concerning the existence, non-existence or probability of the disorder in a patient. It further includes the provision of information concerning the type or classification of the disorder or of symptoms which are or may be experienced in connection with it. This may include, for example, diagnosis of the severity of the disorder. It encompasses prognosis of the medical course of the disorder, for example its duration, severity and the course of progression from MCI to Alzheimer's disease.

Currently disease status is assessed by duration of disease from inception to present (longer duration equals more severe disease) and clinical assessment measures. These assessment measures include clinical tests for memory and other cognitions, clinical tests for function (abilities of daily living) and clinical assessments of global severity. Trials of potential therapies in AD are currently evaluated against these measures. The FDA and other medicines approval bodies require as part of these assessments measures of both cognition and global function. The Global Dementia Scale is one such measure of global function. It is assessed by later assessment of severity including cognition and function against a standardised set of severity criteria.

The term "alleviate", as used herein, in relation to Alzheimer's disease means any form of reducing one or more undesired symptoms or effects thereof. Any amelioration of Alzheimer's disease of the patient falls within the term "alleviation". Amelioration may also include slowing down the progression of the disease.

As used herein "assessing" AD includes the provision of information concerning the type or classification of the disease or of symptoms which are or may be experienced in connection with it. This specifically includes prognosis of the medical course of the disease, for example its duration, severity and the course and rate of progression from e.g. MCI or pre-symptomatic AD to clinical AD. This also includes prognosis of AD-associated brain pathology such as fibrillar amyloid burden, cortical and hippocampal atrophy and accumulation of neurofibrillary tangles. The assessment may be of an aggressive form of AD and/or a poor prognosis.

As used herein "biological sample" refers to any biological liquid, cellular or tissue sample isolated or obtained from the subject. In accordance with the present invention the "protein-containing sample" may be any biological sample as defined herein. The biological sample may, in certain cases, comprise blood plasma, blood cells, serum, saliva, urine, cerebro-spinal fluid (CSF) or a tissue biopsy. The biological sample may have been stored (e.g. frozen) and/or processed (e.g. to remove cellular debris or contaminants) prior to determining the amount (e.g. concentration) of the at least one protein isoform and/or glycoform in question that is found in the sample.

### Exemplary glycoform analysis - clusterin

Clusterin (Apolipoprotein J; SP-40,40; TRPM-2; SGP-2; pADHC-9; CLJ; T64; GP III; XIP8) is a highly conserved disulfide-linked secreted heterodimeric glycoprotein of 75-80 kDa but truncated forms targeted to nucleus have also been identified. The protein is constitutively secreted by a number of cell types including epithelial and neuronal cells and is a major protein in physiological fluids including plasma, milk, urine, cerebrospinal fluid and semen.

Preferably, clusterin comprises or consists of an amino acid sequence having at least 70%, 80%, 90%, 95%, 99% or 100% identity to the human clusterin sequence disclosed in UniProt Accession No. P10909, sequence version 1 and GI No. 116533 (SEQ ID NO: 1), calculated over the full length of said human clusterin sequence; or a fragment thereof comprising at least 5, 10, 15, 20, 30, 50, 100, 150, 200, 250, 300, 350, 400, 425 or 449 contiguous amino acids.

Expression of the clusterin gene is significantly elevated in Alzheimer's disease (AD) brain (May et *al.,* 1990) and levels of plasma clusterin have also been shown to correlate with AD progression (Thambisetty et *al.,* 2010). The inventors have previously identified several plasma clusterin isoforms as candidate biomarkers for AD using 2-dimensional gel electrophoresis (2DE).

However, the use of immunoassays and unmodified peptides in selected reaction monitoring (SRM) experiments did not fully replicate the regulation seen in 2DE. The inventors hypothesised that this disconnect is perhaps due to alterations in specific post-translational events that were not being replicated in the validation studies. Clusterin is a highly-glycosylated secreted protein and because glycosylation plays an important role in physiological functions of clusterin (Stuart et *al.,* 2007) the inventors proposed that the detailed profiling of plasma clusterin and comparison of glycosylation profiles observed in distinct clinically classified subjects, for example patients with low or high atrophy of the hippocampus, may reveal more potent biomarker isoforms.

Guided by the observations relating to the clusterin glycoforms, which demonstrate a β-N-acetyl-glucosaminidase activity in plasma, the inventors also devised a novel assay using a defined substrate to measure this specific activity.

### Example 1. Gel Electrophoresis Analysis of Plasma Clusterin Isoforms

### Methods

Human clusterin, was enriched by immunoprecipitation (IP) from albumin/IgG-depleted plasma, using a monoclonal anti-clusterin antibody (Millipore). Immunoprecipitated proteins were first analysed by Western blotting as a quality control, then separated by either two-dimensional electrophoresis (2DE) or SDS-PAGE. The spots and single band (#3) of interest were excised, reduced, alkylated and digested in-gel with trypsin prior to analysis by mass spectrometry (MS). Samples were analysed via LC-MS/MS using nanoflow reverse phase chromatography (EASY-nLC II, ThermoFisher Scientific) and a Top20 collision induced dissociation (CID) method (Orbitrap Velos, ThermoFisher Scientific). Glycopeptides were manually identified by the presence of glycan-specific oxonium ion fragments, m/z 204.08 for N-acetylhexosamine, [HexNAc]⁺, m/z 366.14 for hexose-N-acetylhexosamine, [Hex-HexNAc]⁺, and m/z 657.24 for N-acetylneuraminic acid-hexose-N-acetylhexosamine [NeuAc-Hex-HexNAc]⁺ in the MS/MS spectra.

### Results

### 2DE spots

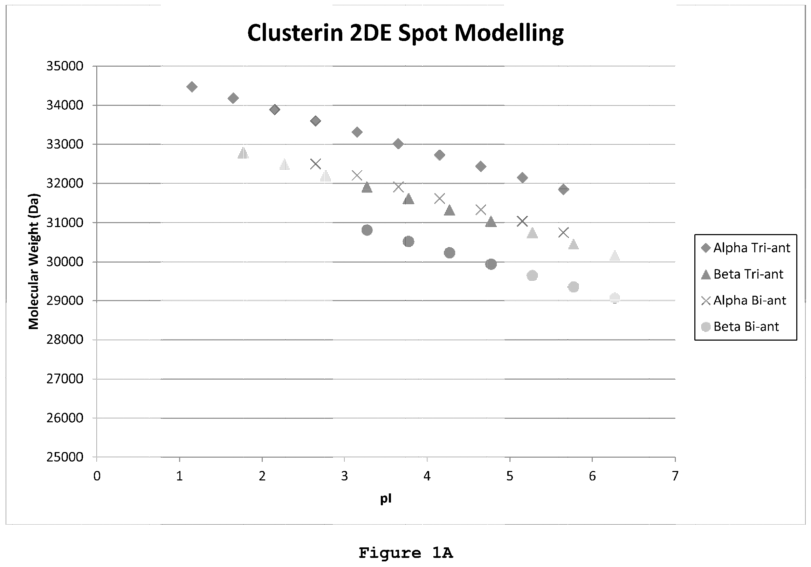

Initially, mathematical modelling was used to create an artificial map of the various clusterin glycoforms for the separate alpha and beta chains (Figure 1A). Further refinement of this approach was used to classify individual clusterin related glycoforms using simple (x, y) coordinates. In this way, the inventors were able to predict the content of the individual 2DE spots, and demonstrate that discrete coordinates are shared by multiple glycoforms. Hence, 2DE spots are likely to be composite mixtures containing several glycoforms. This information was then used to aid the interpretation of complex LC/MS/MS data, which subsequently provided some rather useful insights.

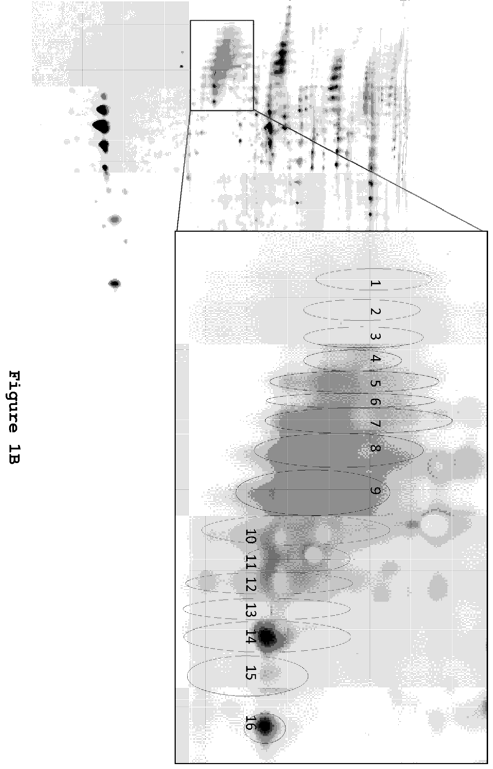

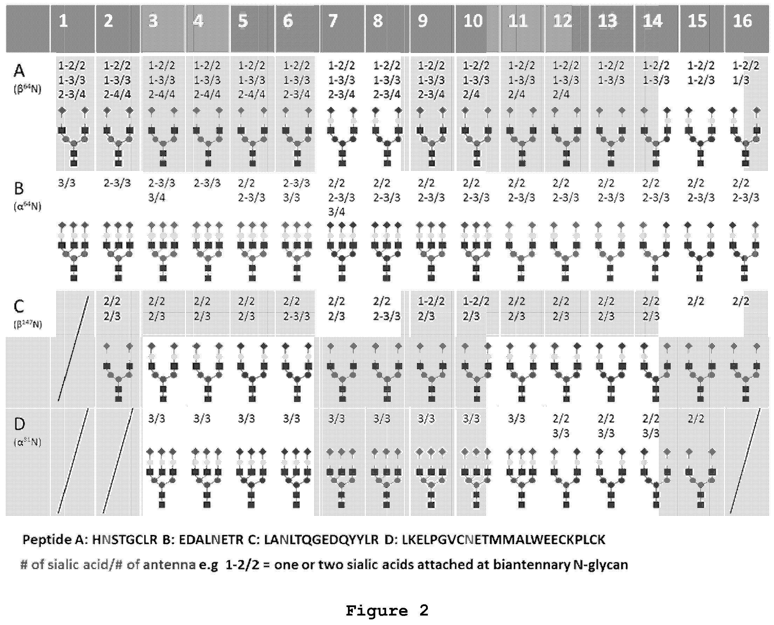

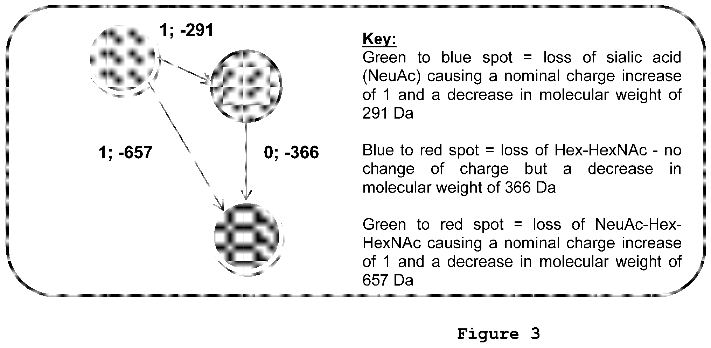

Firstly, it became apparent that the major components within each of the 2DE spots were, without exception, always fully sialylated forms, being either tetra, tri or biantennary structures (Figure 1B). This was surprising because it had originally been predicted that differences in sialic acid were the primary cause of the separation of distinct forms during electrophoresis, with loss of 291Da concurrent with a decreasing charge thus resulting in a shift towards a more basic iso-electric point. However, the LC/MS/MS results (Figure 2) indicated a trend towards lower number of antennae and this suggested the successive removal of whole antennae as a more pronounced effect on the location of the 2DE spots. A basic vector was devised to illustrate this phenomena (Figure 3) and it transpires that the detected clusterin glycoforms actually now indicate evidence to support both the removal of sialic acids alone as well as removal of full antennae suggesting distinct neurominidase and β-N-acetyl-glucosaminidase activity respectively.

### Example 2 - Design of a substrate assay to measure specific n-acetyl-glucosaminidase activity in plasma

The results of glycan analysis of 16 different clusterin isoforms visible on 2-dimensional gel electrophoresis showed a sequential removal of sialic acids and entire antennae. Several of the truncated glycoforms appeared to correlate with clusterin protein spots previously identified as candidate biomarkers of AD and MCI. Until now no detailed analysis of glycosylation of these clusterin isoforms has been performed and it was surprising to discover that the majority of the disease associated modification in plasma clusterin could be accounted for by the activity of a single glycosidase, namely β-N-acetyl-glucosaminidase.

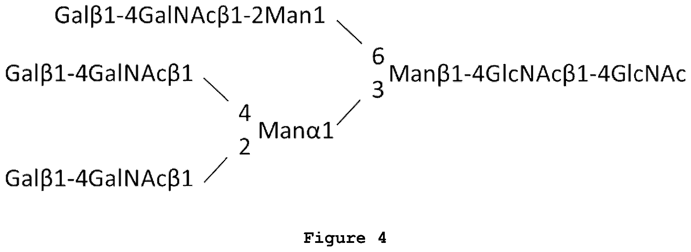

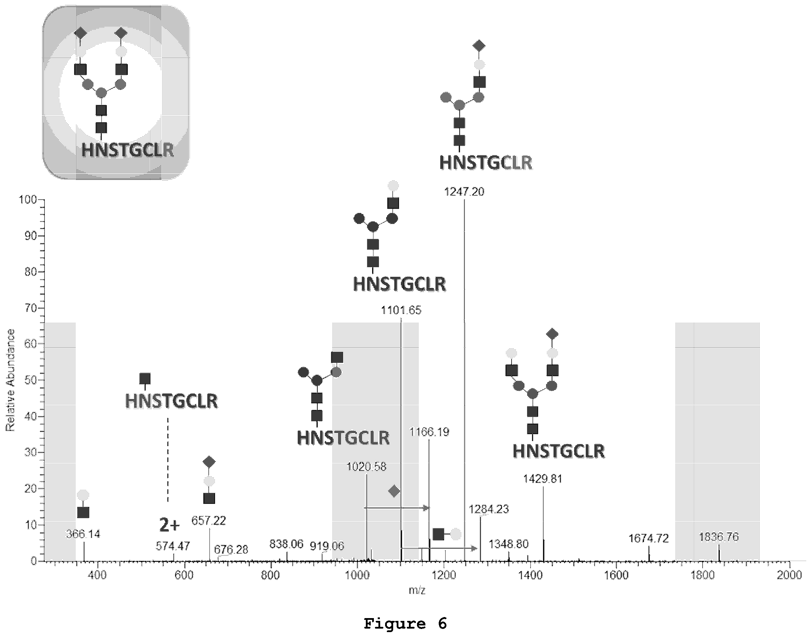

The inventors thus set up a specific assay method to determine the activity of β-N-acetyl-glucosaminidase in tissue or bodily fluid samples taken from subjects suspected of having, or previously diagnosed with MCI, AD or other dementia. The artificial glycan NA3 substrate (Figure 4) contains both β1,2 and β1,4 linkages between adjoining β-N-acetyl-glucosamine and mannose subunits and is a preferred substrate to differentiate β1,2 and β1,4 N-acetyl-glucosaminidase activity in plasma. The molecular weight of the substrate is 2006.82Da and an [M+2H]²⁺ ion is detected at m/z 1003.8 using an ESI-TOF mass spectrometer (Figure 6).

NA3 substrate is added to an appropriate sample of tissue or body fluid from a subject suspected of having, or previously diagnosed with dementia to achieve a final concentration of 300 - 1,000 pg/µl and incubated at 37°C for 4-24 hours. The test sample is then centrifuged to remove debris and an aliquot submitted to LC-MS/MS analysis. The measurement of molecular ions corresponding to loss of either two or one antennae indicate β1,2 and β1,4 N- acetyl-glucosaminidase activity respectively.

### Example 3 - Analysis of Immuno-precipitated Clusterin to create a unique glycopeptide reference resource for Clusterin:_The Clusterin GlycoMod database v1.0

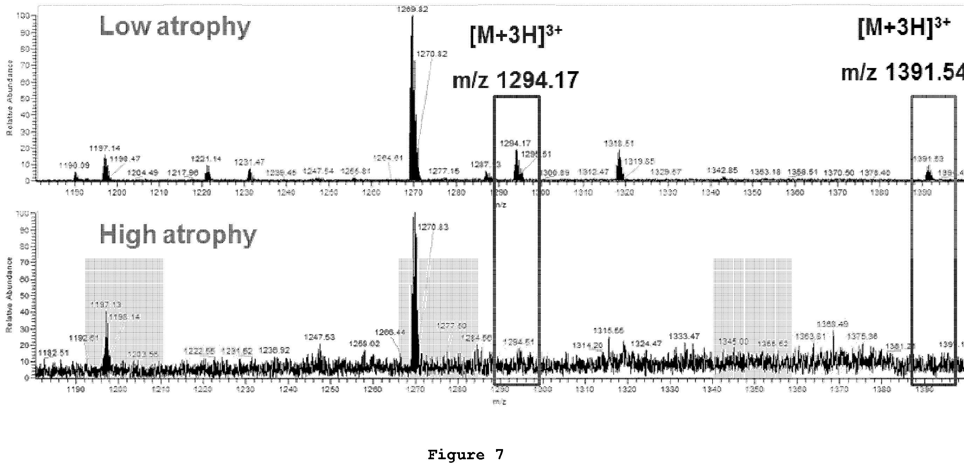

A representative pooled clinical plasma sample was used to develop methodology and to assemble an "observation-based" database containing 41 distinct glycoforms associated with anticipated glycosylation consensus sites within the amino acid sequence. For each glycopeptide the m/z charge state and retention time (RT) of the analyte was tabulated (see Table 1A). Unambiguous annotation of the glycopeptide required the detection of the [Peptide+HexNAc]⁺ fragment ion in the corresponding MS/MS spectra and interpretation of additional fragment ions relating to the sequential dissociation of the individual glycan subunits. An example MS/MS spectrum is shown (Figure 7) and the current iteration of the Clusterin GlycoMod database is provided in Table 1A. An updated iteration of the Clusterin GlycoMod database is provided in Table 1B.

Using immuno-precipitation and LC/MS/MS we have characterised 41 glycopeptides encompassing 5 of 6 anticipated N-linked glycosylation consensus sites in plasma clusterin. In total 41 different N-linked glycopeptides have been characterised and are listed herein. The glycan distribution at these 5 sites was consistent with a CV of <15% (n=3 from two plasma samples) indicating the technical and biological reproducibility of the method.

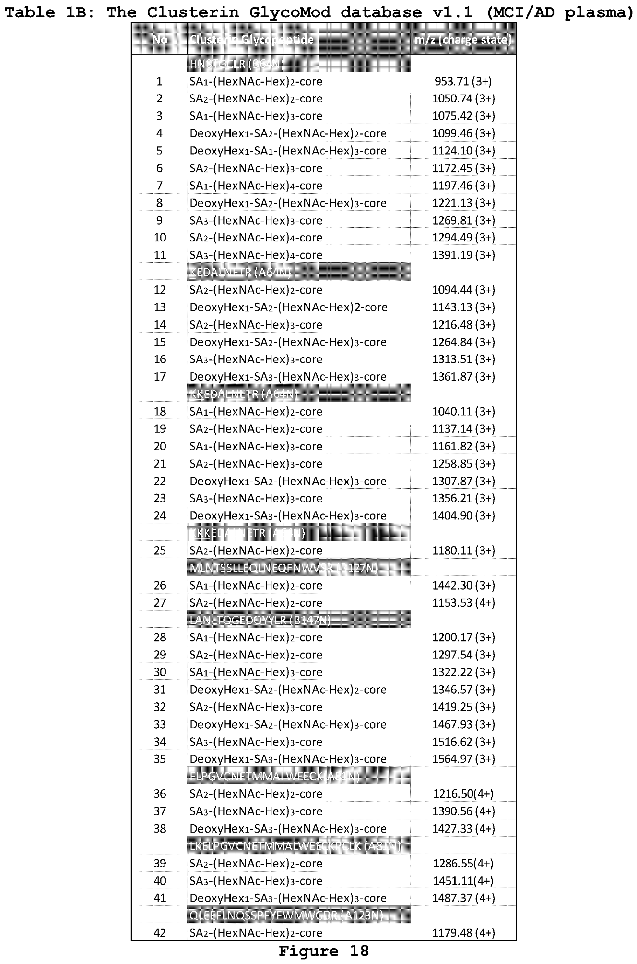

The inventors have previously demonstrated 5 of 6 predicted N-linked glycosylation sites within human plasma clusterin (GlycoMod database v1.0). It would be understood by the skilled practitioner that expansion of the GlycoMod database to cover all N-linked and O-linked sites of all the protein biomarkers is within the scope of the present invention. Indeed, the inventors have subsequently completed mapping of the sixth N-linked site in human clusterin as set out in Table 1B (Figure 18), showing clusterin glycopeptides of version 1.1 of the clusterin GlycoMod database (MCI/AD plasma).

### Example 4 - Analysis of Immuno-precipitated Clusterin to compare individuals with low and high atrophy

The inventors have identified certain isoforms of clusterin as differentially regulated in the plasma of patients with AD relative to non-demented controls. Furthermore, it has also been shown that certain spots comprising clusterin on 2DE gels correlate with the level of hippocampal atrophy, whilst yet other isoforms correlated with the subsequent rate of disease progression in AD.

The inventors obtained plasma samples from four subjects previously diagnosed with AD who had a low level of hippocampal atrophy and from four subjects previously diagnosed with AD with high hippocampal atrophy. Clusterin was enriched using immunoprecipitation, and subjected to the LC-MS/MS method described above. Surprisingly, they identified that the extent of glycan pruning correlated with hippocampal atrophy. In patients with low levels of hippocampal atrophy there was little evidence of pruning of plasma clusterin. Conversely, plasma clusterin from subjects with high levels of hippocampal atrophy was typically pruned to remove one or more complete antennae within the N-linked glycans.

As an example, two sialylated forms of the tetra-antennary glycopeptide **HN*STGCLR** (SEQ ID NO: 2) are observed as triply charged molecular ions at m/z 1391 and m/z 1294.17 but only in individuals with low atrophy (Figure 7). These moieties are therefore potential alternative biomarkers concordant with the extent of hippocampal atrophy.

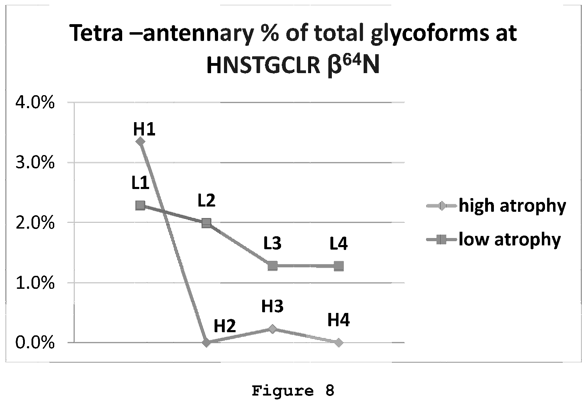

Using data from all N-linked glycans monitored by the LC-MS/MS method the inventors saw a consistent reduction in the level of tetra-antennary glycans in subjects with high levels of hippocampal atrophy compared to those with low levels. Based on the total glycoform signal for the N-linked glycosylation site on the tryptic peptide HN*STGCLR (SEQ ID NO: 2) of the clusterin beta chain (Figure 8).

### Example 5 - Validation Analysis of Immuno-precipitated Clusterin to compare individuals with low and high atrophy

Having identified that changes in Clusterin glycosylation patterns correlate to the extent of atrophy within a small cohort of clinical samples we performed a further validation study on an additional cohort of Alzheimer's disease patients with known levels of hippocampal atrophy. Additional bioinformatics approaches were also assessed for their impact on class segregation based on glycoform profiles and a new, higher sensitivity mass spectrometer was employed in the expectation of identifying additional diagnostic glycoforms of clusterin. To ensure correlation with earlier data, samples from the original 4 x 4 cohort (Discovery Cohort) used in Example 4 were re-analysed using the new methods alongside a separate cohort of 20 new samples from AD (n=10) and matched controls (n=10)(Replication Cohort). All sample details are provided in Table 2.

### Sample Cohort Details

**Table 2. Sample details associated with 4 vs 4 and 10 vs 10 cohorts**

| Study ID | Disease group | Gender | Age | Mean Clusterin | Mean hippocampus (x10e-6) | Atrophy |
|---|---|---|---|---|---|---|
| 4.1 | AD | Female | 82 | 87 | 135 | High |
| 4.2 | MCI | Male | 79 | 93 | 264 | Low |
| 4.3 | MCI | Male | 72 | 90 | 274 | Low |
| 4.4 | MCI | Male | 75 | 90 | 264 | Low |
| 4.5 | AD | ND | ND | 153 | 161 | High |
| 4.6 | AD | Female | 78 | 84 | 164.5 | High |
| 4.7 | AD | Male | 69 | 90 | 106.5 | High |
| 4.8 | MCI | Female | 71 | 114 | 307.5 | Low |
| 10.1 | AD | Male | 79 | 154.32 | 111.5 | High |
| 10.2 | AD | Female | 76 | 337.7 | 124.0 | High |
| 10.3 | AD | Male | 77 | 422.11 | 125.5 | High |
| 10.4 | AD | Male | 69 | 252.19 | 233.0 | Low |
| 10.5 | AD | Female | 87 | 322.05 | 238.0 | Low |
| 10.6 | AD | Male | 71 | 289.22 | 228.0 | Low |
| 10.7 | AD | Female | 70 | 253.82 | 234.2 | Low |
| 10.8 | AD | Male | 70 | 303.13 | 0.0 | High |
| 10.9 | AD | Female | 83 | 530.31 | 136.5 | High |
| 10.10 | AD | Female | 65 | 497.37 | 227.1 | Low |
| 10.11 | AD | Female | 77 | 404.6 | 235.5 | Low |
| 10.12 | AD | Female | 76 | 241.56 | 99.6 | High |
| 10.13 | AD | Male | 76 | 300.21 | 109.5 | High |
| 10.14 | AD | Female | 67 | 323.54 | 135.0 | High |
| 10.15 | AD | Female | 72 | 280.5 | 228.0 | Low |
| 10.16 | AD | Female | 63 | 309.15 | 244.7 | Low |
| 10.17 | AD | Male | 83 | 423.18 | 127.0 | High |
| 10.18 | AD | Female | 71 | 7147.63 | 237.4 | Low |
| 10.19 | AD | Female | 68 | 307.14 | 140.0 | High |
| 10.20 | AD | Male | 79 | 351.02 | 237.5 | Low |

### Methods

Clusterin was enriched from each sample, as described above. The relevant protein band was excised, reduced, alkylated, and digested with Trypsin. After clean up, the clusterin digests were split into two aliquots and each tested by nanoflow high performance liquid chromatography and Orbitrap Velos Pro or ultra-high performance liquid chromatography and Orbitrap Fusion Tribrid LC-MS/MS systems (all equipment from Thermo Scientific, Hemel Hempstead, UK). Data were ostensibly similar but, as expected, more glycosylated clusterin peptides were identified on the Fusion and so all subsequent analysis was performed on the Fusion dataset.

### Bioinformatics

Mass spectrometer raw data were processed using Proteome Discoverer software (Thermo Scientific). Ion intensities for the glycosylated clusterin peptides and their fragments described in Tables 1A and 1B were exported into an Excel (Microsoft Corp) spreadsheet. We employed a sum scaling technique to normalise the data and calculated significance values (p) for each glycopeptide by comparing the median values between the low and high atrophy groups in the Discovery Cohort, Replication Cohort and a combined analysis of both Cohorts as a single group. Student's T test was used to identify peptide-associated glycoforms that change significantly between high and low atrophy, resulting in one-tailed p-values for each glycopeptide (see Tables 3A, 3B and 3C).

### Results

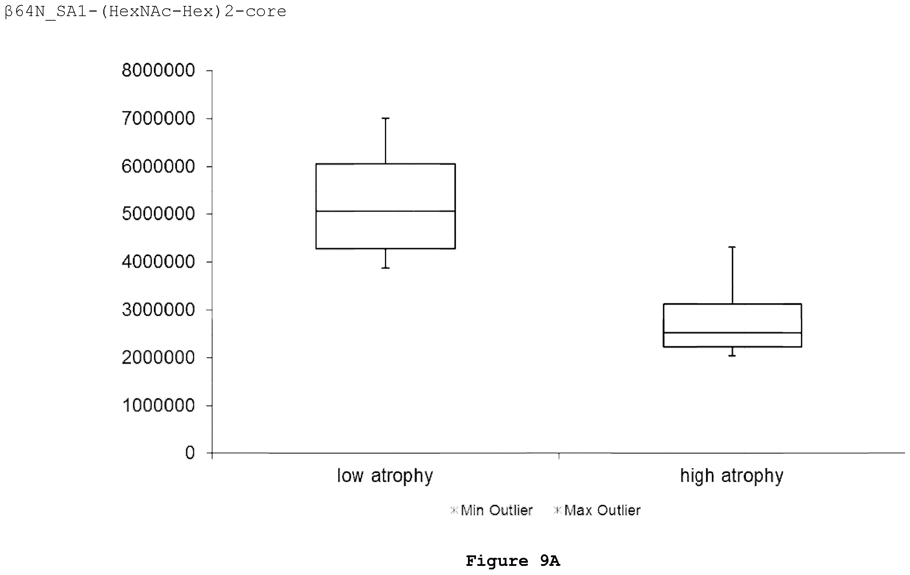

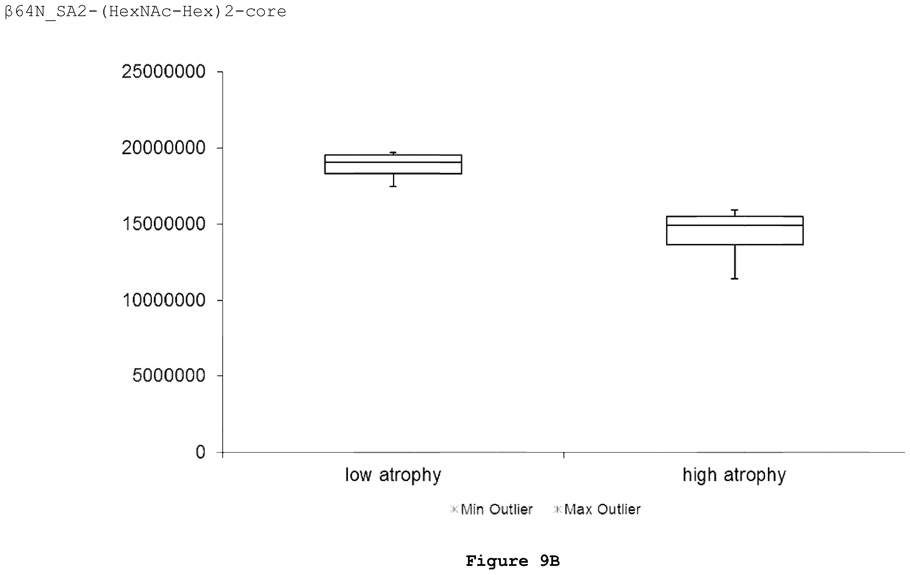

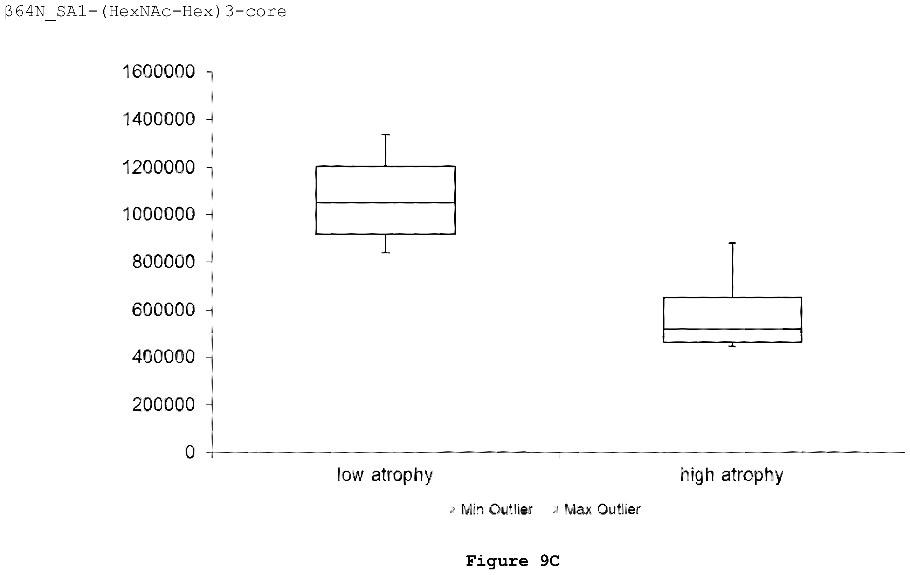

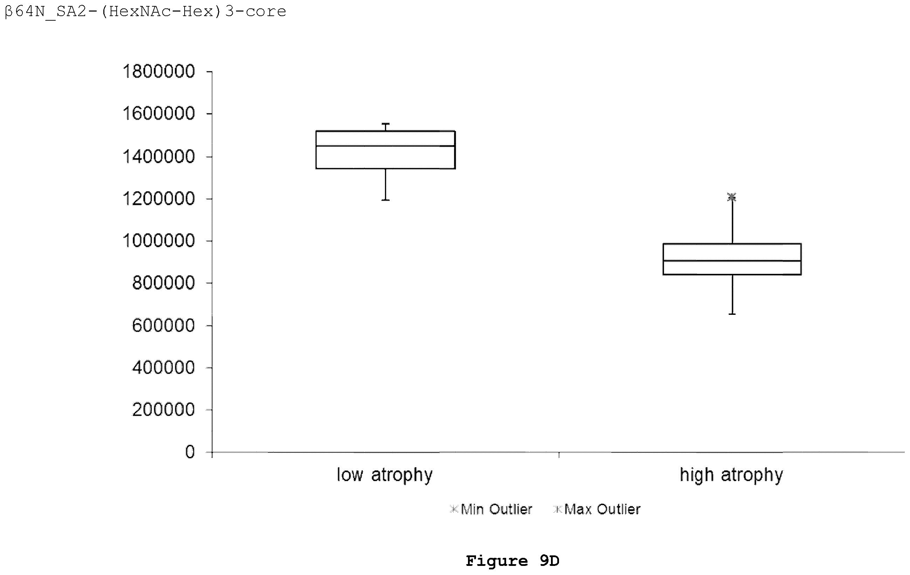

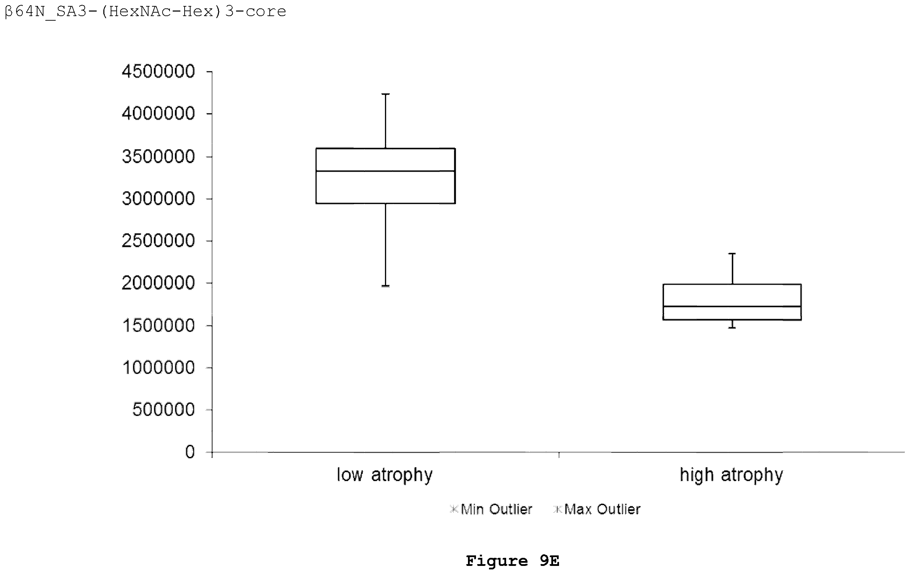

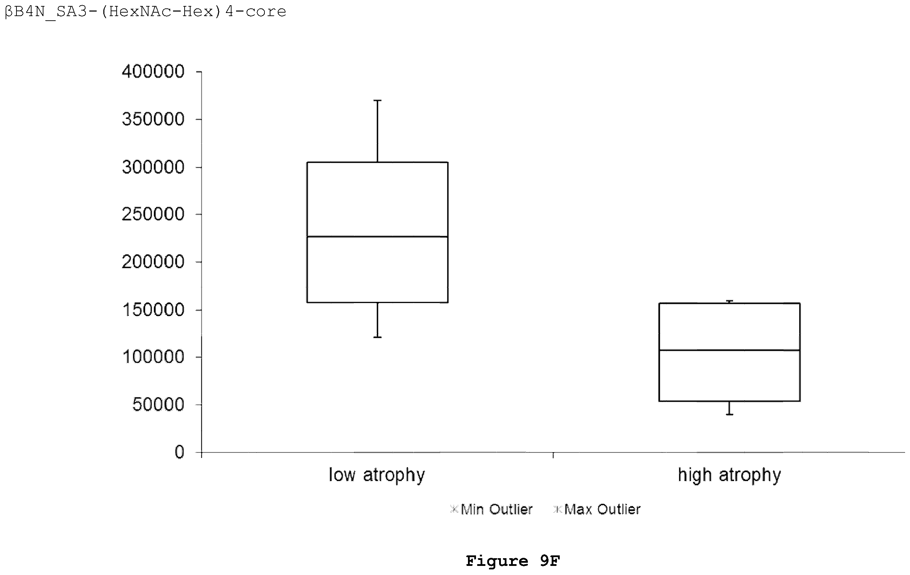

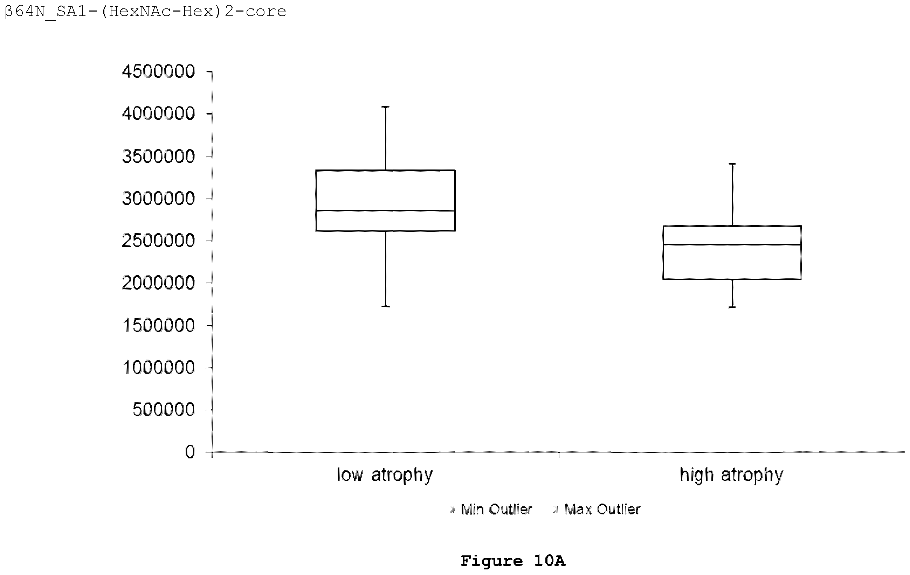

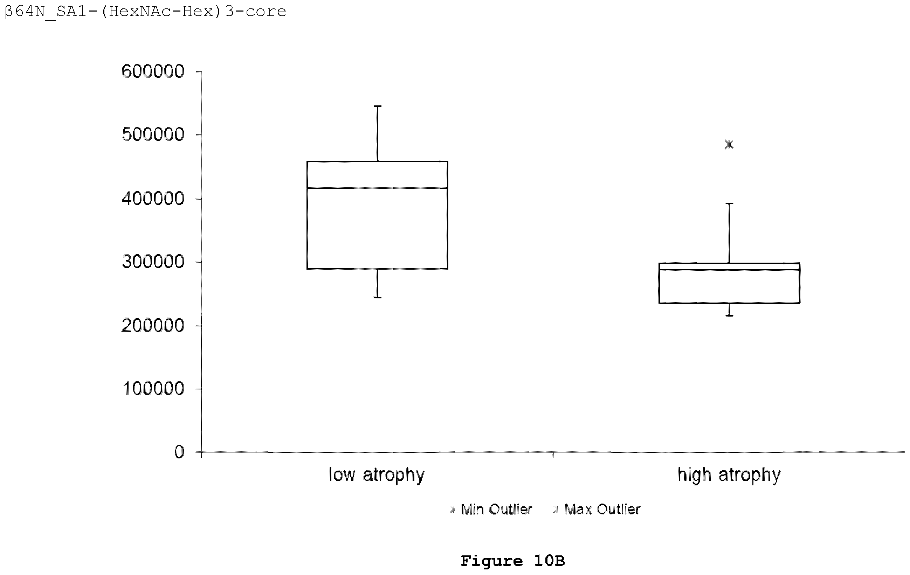

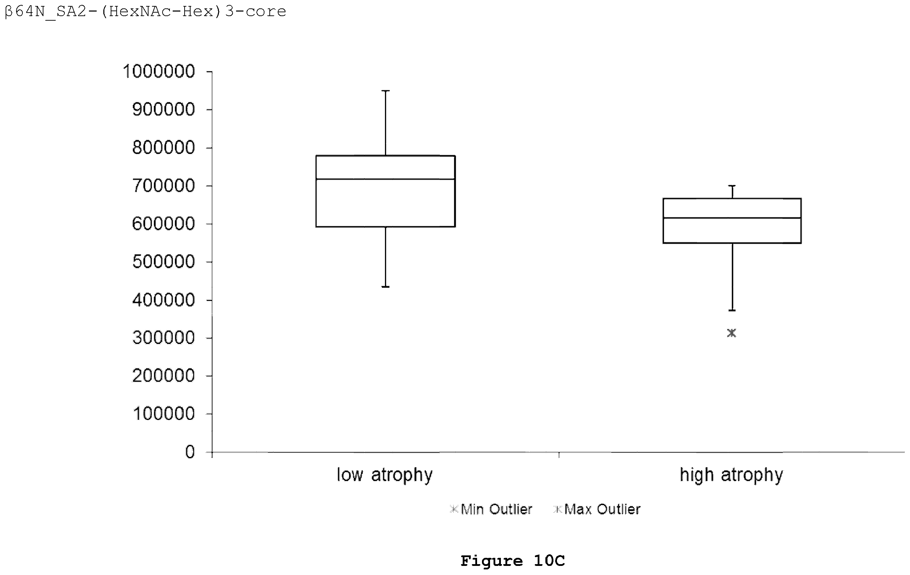

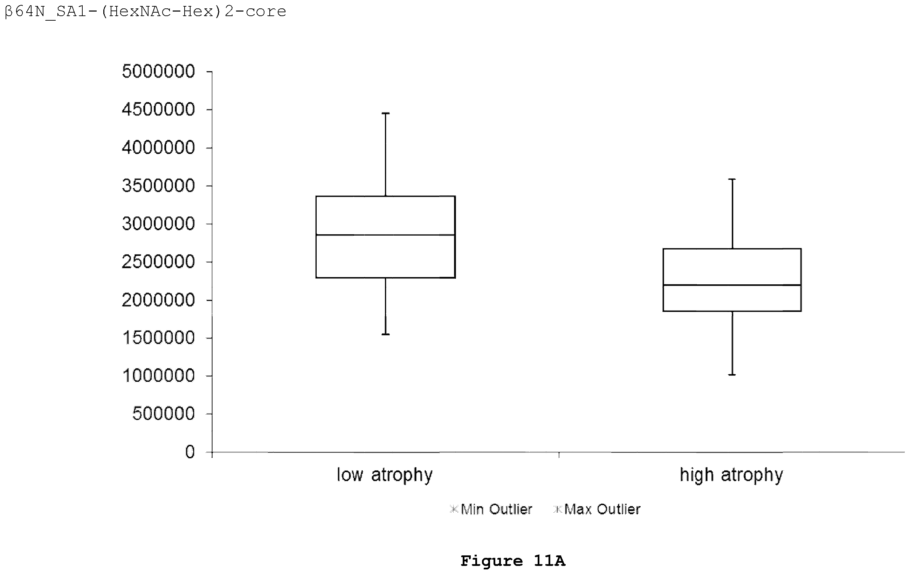

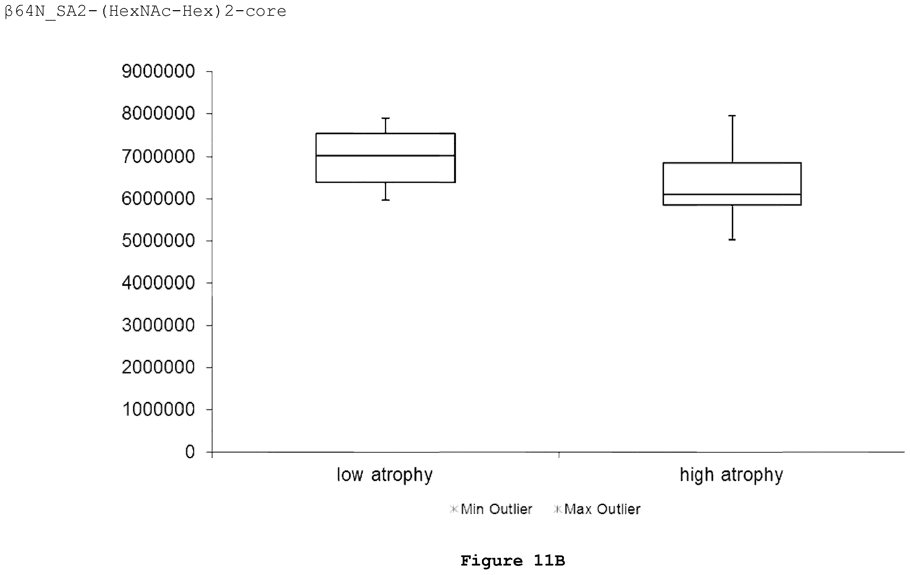

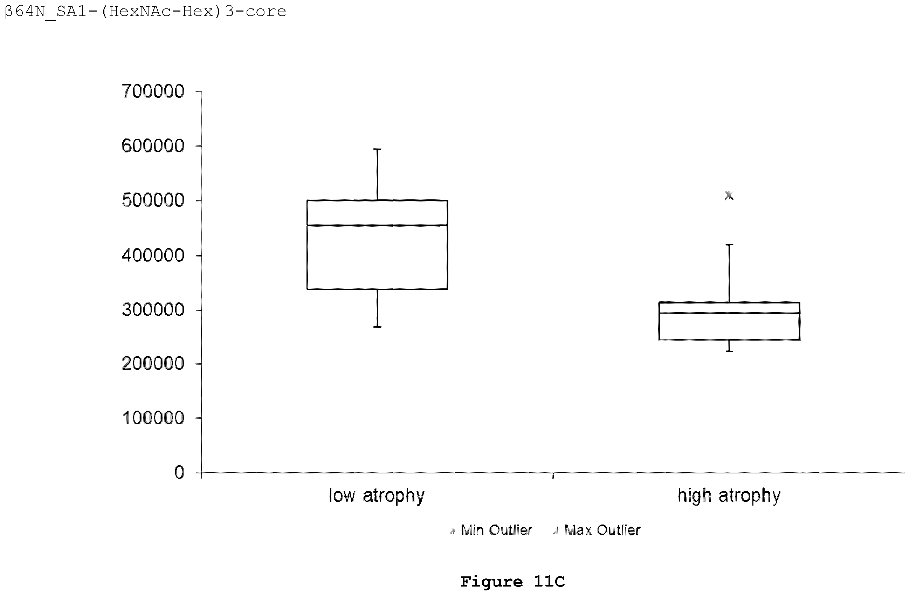

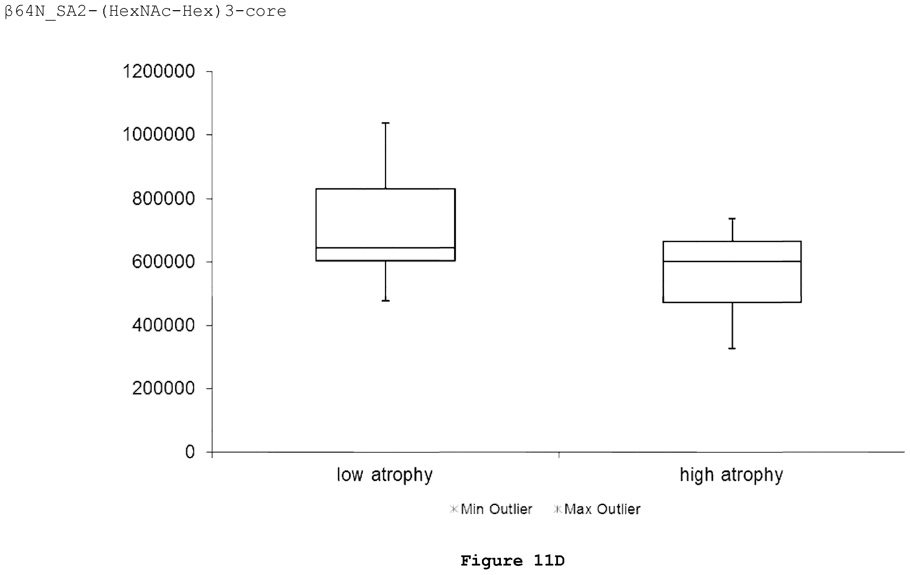

Using our IP-LC/MS/MS workflow on the Orbitrap Fusion Tribrid we were able to extend coverage to all six known N-glycosylation sites of clusterin: α64N, α81N, α123N, β64N, β127N, and β147N. By monitoring the glycan specific fragments we were also able to assign various antennary structures at all six sites and to perform relative quantification based on total ion counts. In total 42 different glycan structures were detected. Whilst most glycosylation sites showed no regulation in glycan structures between high and low levels of hippocampal atrophy, two sites - β64N and β147N - showed significant regulations between the clinical groups. The specific glycan structures showing significant (p ≤ 0.05) changes between the clinical groups in the Discovery, Replication and combined Cohort analyses are indicated in Table 3A, 3B, and 3C respectively. Box plots for each glycopeptide were created to illustrate the separation achieved between the two groups (Figures 9-11).

Interestingly, six glycoforms at β64N glycosylation site HN*STGCLR (SEQ ID NO: 2) were found significantly decreased in the 4 high atrophy samples (Alzheimer's)compared to the 4 low atrophy samples (mild cognitive impairment) of the Discovery Cohort when measured on the Orbitrap Fusion. This included the sialylated forms of the tetra-antennary glycopeptide observed as triply charged molecular ions at m/z 1391.54 which was consistent with the previous Velos data analysis, confirming the robustness of this glycoform as a diagnostic marker to differentiate mild cognitive impairment from Alzheimer's disease when measured on a different LC-MS/MS platform.

In the larger replication cohort, three glycoforms of β64N glycopeptides were significantly reduced in high atrophy samples. These include the SA1-(HexNAc-Hex)2, SA1-(HexNAc-Hex)3 and SA2-(HexNAc-Hex)3 glycoforms seen at m/z 953.71, 1075.42, 1172.45 in the spectra. As all of these glycoforms were also seen reduced in high atrophy patients in the Discovery Cohort this further supports their utility as prognostic biomarkers in patients with confirmed Alzheimer's disease.

When the results of the two cohorts were combined we again, saw that changes in glycoforms found at site β64N correlated with atrophy, with four glycoforms significantly reduced over high atrophy, e.g. SA1-(HexNAc-Hex)2, SA2-(HexNAc-Hex)2, SA1-(HexNAc-Hex)3, and SA2-(HexNAc-Hex)3 at m/z 953.71, 1050.74, 1075.42, and 1172.45 respectively.

**Table 3A Significant changes in Clusterin glycopeptides (4 vs 4)**

| M/Z (3+) | COMPOSITION | P-VALUE | SITE |
|---|---|---|---|
| 953.71 | SA1-(HexNAc-Hex)2-core | 0.016 | β64N |
| 1050.74 | SA2-(HexNAc-Hex)2-core | 0.003 | β64N |
| 1075.42 | SA1-(HexNAc-Hex)3-core | 0.009 | β64N |
| 1172.45 | SA2-(HexNAc-Hex)3-core | 0.006 | β64N |
| 1269.49 | SA3-(HexNAc-Hex)3-core | 0.017 | β64N |
| 1391.53 | SA3-(HexNAc-Hex)4-core | 0.043 | β64N |
| 1297.54 | SA2-(HexNAc-Hex)2-core | 0.044 | β147N |
| 1356.21 | SA3-(HexNAc-Hex)3-core | 0.044 | α64N |

**Table 3B Significant changes in Clusterin glycopeptides (9 vs 10)**

| M/Z (3+) | COMPOSITION | P-VALUE | SITE |
|---|---|---|---|
| 953.71 | SA1-(HexNAc-Hex)2-core | 0.035 | β64N |
| 1075.42 | SA1-(HexNAc-Hex)3-core | 0.019 | β64N |
| 1172.45 | SA2-(HexNAc-Hex)3-core | 0.043 | β64N |
| 1297.54 | SA2-(HexNAc-Hex)2-core | 0.044 | β147N |
| 1137.14 | SA2-(HexNAc-Hex)2-core | 0.016 | α64N |
| 1356.21 | SA3-(HexNAc-Hex)3-core | 0.007 | α64N |

**Table 3C Significant changes in Clusterin glycopeptides (combined 13 vs 14)**

| M/Z (3+) | COMPOSITION | P-VALUE | SITE |
|---|---|---|---|
| 953.71 | SA1-(HexNAc-Hex)2-core | 0.022 | β64N |
| 1050.74 | SA2-(HexNAc-Hex)2-core | 0.022 | β64N |
| 1075.42 | SA1-(HexNAc-Hex)3-core | 0.001 | β64N |
| 1172.45 | SA2-(HexNAc-Hex)3-core | 0.019 | β64N |

### Conclusion

Use of the Orbitrap Fusion increased total glycoform coverage from 4 to 6 N-linked sites. Several β64N site glycoforms are significantly reduced in plasma of patients with Alzheimer's disease compared to individuals with mild cognitive impairment. Four of these glycoforms are also reduced in Alzheimer's patients with high levels of hippocampal atrophy. In combination this confirms the utility of clusterin isoforms as diagnostic and prognostic markers for Alzheimer's disease.

### Example 6 - Development and preliminary testing of a Selective Reaction Monitoring (SRM) method for 8 glycoforms of clusterin in human plasma

In readiness for higher throughput measurements within much larger numbers of clinical samples, we have also developed a targeted Selective Reaction Monitoring (SRM) method to measure specific glycopeptides of Clusterin. This newly established TSQ-SRM workflow used eight glycoforms of Clusterin β64N glycopeptides as precursors, and two glycan-specific oxonium ion fragments at m/z 366.14 and m/z 657.24 as transitions (see Table 4). Additionally, the peptide ion at m/z 574.56 representing [HN*STGCLR]²⁺ (SEQ ID NO: 2) where N* = Asparagine residue + HexNac, was included to serve as the third transition ion providing confirmation of site-specific information. Details of each monitored transition is provided in Table 4.

**Table 4. Glyco-SRM method of TSQ analysis.**

| # | Parent | Product | SRM collision energy | Start Time | Stop Time | Polarity | Trigger | Reference |
|---|---|---|---|---|---|---|---|---|
| 1 | 953.712 | 366.140 | 30 | 0 | 30 | + | 100 | No |
| 2 | 953.712 | 574.556 | 30 | 0 | 30 | + | 100 | No |
| 3 | 953.712 | 657.235 | 30 | 0 | 30 | + | 100 | No |
| 4 | 1050.744 | 366.140 | 33 | 0 | 30 | + | 100 | No |
| 5 | 1050.744 | 574.556 | 33 | 0 | 30 | + | 100 | No |
| 6 | 1050.744 | 657.235 | 33 | 0 | 30 | + | 100 | No |
| 7 | 1075.423 | 366.140 | 34 | 0 | 30 | + | 100 | No |
| 8 | 1075.423 | 574.556 | 34 | 0 | 30 | + | 100 | No |
| 9 | 1075.423 | 657.235 | 34 | 0 | 30 | + | 100 | No |
| 10 | 1172.454 | 366.140 | 37 | 0 | 30 | + | 100 | No |
| 11 | 1172.454 | 574.556 | 37 | 0 | 30 | + | 100 | No |
| 12 | 1172.454 | 657.235 | 37 | 0 | 30 | + | 100 | No |
| 13 | 1197.134 | 366.140 | 38 | 0 | 30 | + | 100 | No |
| 14 | 1197.134 | 574.556 | 38 | 0 | 30 | + | 100 | No |
| 15 | 1197.134 | 657.235 | 38 | 0 | 30 | + | 100 | No |
| 16 | 1269.487 | 366.140 | 41 | 0 | 30 | + | 100 | No |
| 17 | 1269.487 | 574.556 | 41 | 0 | 30 | + | 100 | No |
| 18 | 1269.487 | 657.235 | 41 | 0 | 30 | + | 100 | No |
| 19 | 1294.165 | 366.140 | 42 | 0 | 30 | + | 100 | No |
| 20 | 1294.165 | 574.556 | 42 | 0 | 30 | + | 100 | No |
| 21 | 1294.165 | 657.235 | 42 | 0 | 30 | + | 100 | No |
| 22 | 1391.532 | 366.140 | 45 | 0 | 30 | + | 100 | No |
| 23 | 1391.532 | 574.556 | 45 | 0 | 30 | + | 100 | No |
| 24 | 1391.532 | 657.235 | 45 | 0 | 30 | + | 100 | No |

In previous studies (data not shown), we were able to extract clusterin glycopeptides from human serum without prior immunoprecipitation. Given the potential sensitivity gains offered by SRM methods we followed a more straightforward geLC method for clusterin enrichment which would be more compatible with high throughput analysis such as would be required for a clinical diagnostic.

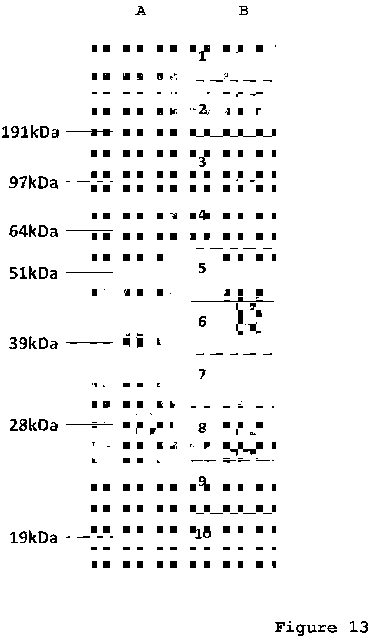

Initially, to identify the location of clusterin in a one dimensional SDS-Polyacrylamide gel electrophoresis (SDS-PAGE) experiment normal human plasma (Dade-Behring, Germany)was depleted of albumin and IgG and proteins extracted in Laemmli buffer and subjected to SDS-PAGE. Figure 13 shows the Gel-10 analysis of albumin/IgG-depleted plasma. All ten bands were excised, reduced, alkylated, and trypsin-digested prior to MS analysis on an Orbitrap Velos Pro. Glycopeptides of clusterin were identified in the tryptic digests of bands #5, #6, and #7 (data not shown)with the majority seen in band #7 with 45% of peptide coverage.

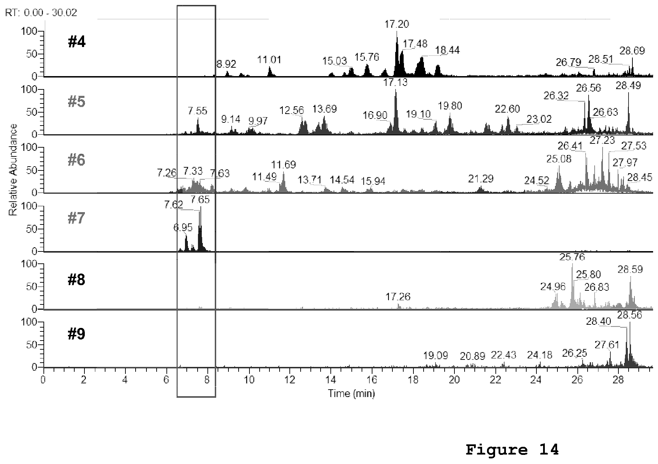

All ten tryptic-digested gel bands were also submitted for analysis using the newly-developed clusterin glycoform SRM method to confirm the suitability of the geLC method for sample preparation. Figure 14 shows Clusterin glycopeptides were found in the band #5, #6 and #7, and majority of Clusterin was identified at band #7, suggesting these glyco-SRM results were consistent with the previous Orbitrap Velos Pro discovery data.

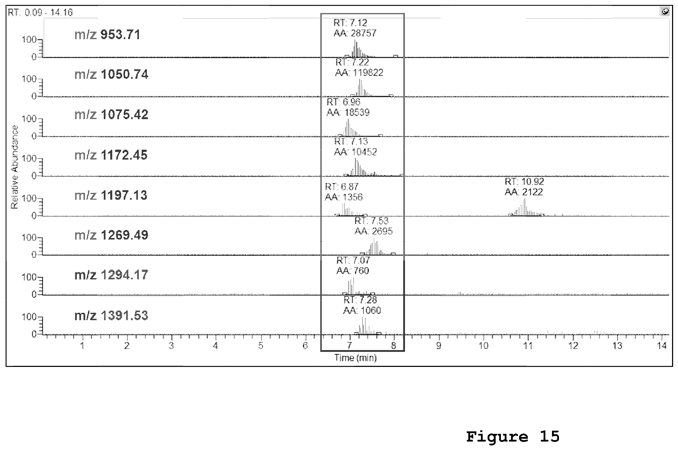

When the SRM data files were examined for the extracted ion chromatograms (XIC) of eight β64N glycopeptide precursors (Figure 15), we were able to determine that the majority of them eluted between 7-8 minutes. Precise identification of elution time allows subsequent scheduling of SRM or adjustment of elution buffers to improve separation of closely related species if more complex methods should be developed subsequently.

It is a particular advantage of the present method that the integrated peak area for each monitored species can be used for label-free quantification using a sum-scaled approach. Furthermore, since this Gel10-glyco-SRM method does not require immunoprecipitation and Clusterin glycopeptides can be detected within 30 minutes by TSQ, instead of one hour by Orbitrap, this newly-established method provides a more efficient and faster way to verify the potential biomarker glycopeptide of Clusterin. The same method may also be used for clinical assessment of patient samples to aid the diagnosis of MCI and Alzheimer's disease as well as providing prognostic information on the rate of hippocampal atrophy and cognitive decline. It would also be understood that the same SRM method may be applied with little further optimisation to digested human plasma, serum, saliva, urine or cerebrospinal fluid without the need for prior SDS-PAGE separation.

It is also possible to employ the same SRM method for the analysis of clusterin enriched from human plasma by immunoprecitpitation. Thus, to further validate our targeted biomarker glycopeptides, the clusterin glycol-SRM method was applied to evaluate immunoprecipitated clusterin from the Discovery Cohort. As expected, the SRM method gave tighter quantitative results and this improved precision resulted in higher levels of significance for the reduction in specific glycoforms in Alzheimer's patients with higher levels of hippocampal atrophy (Table 5). In total, five of the eight monitored glycopeptides at β64N were significantly reduced in high atrophy cases.

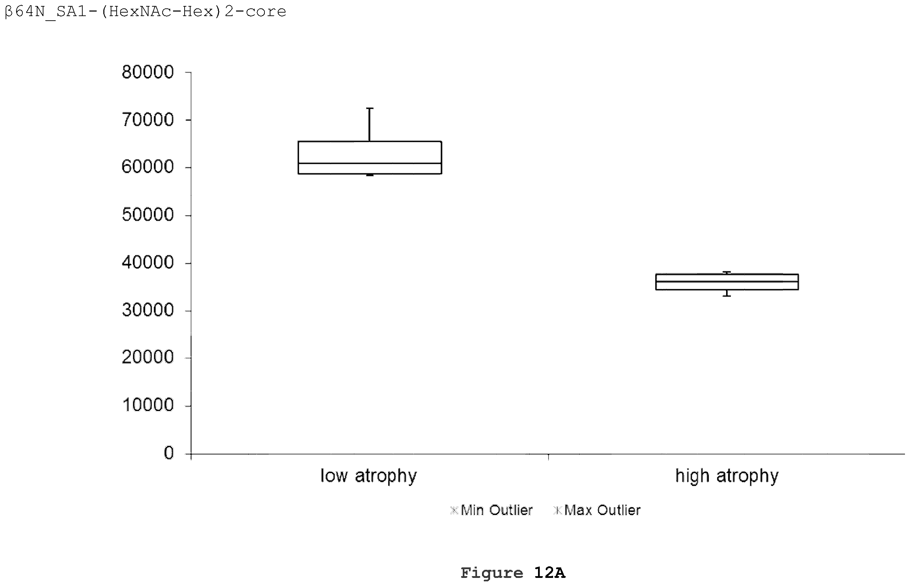

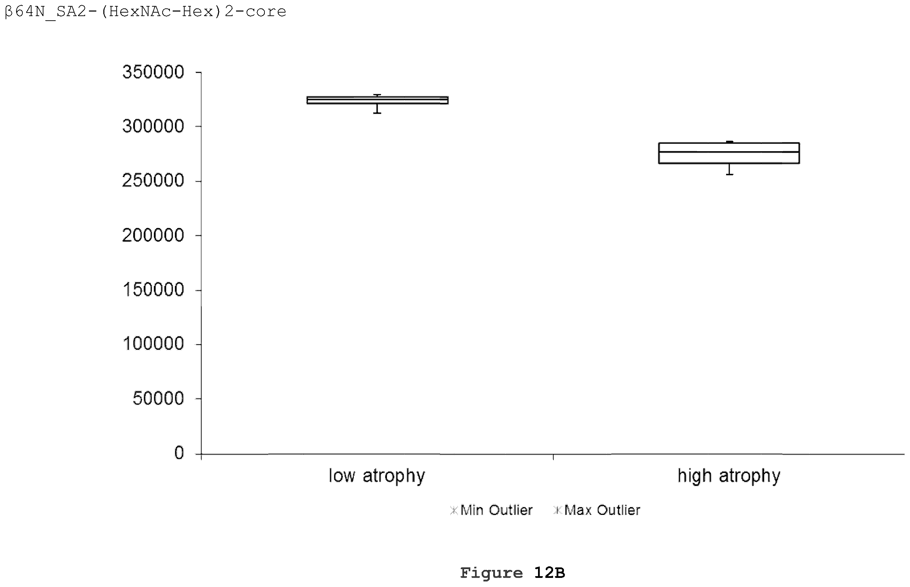

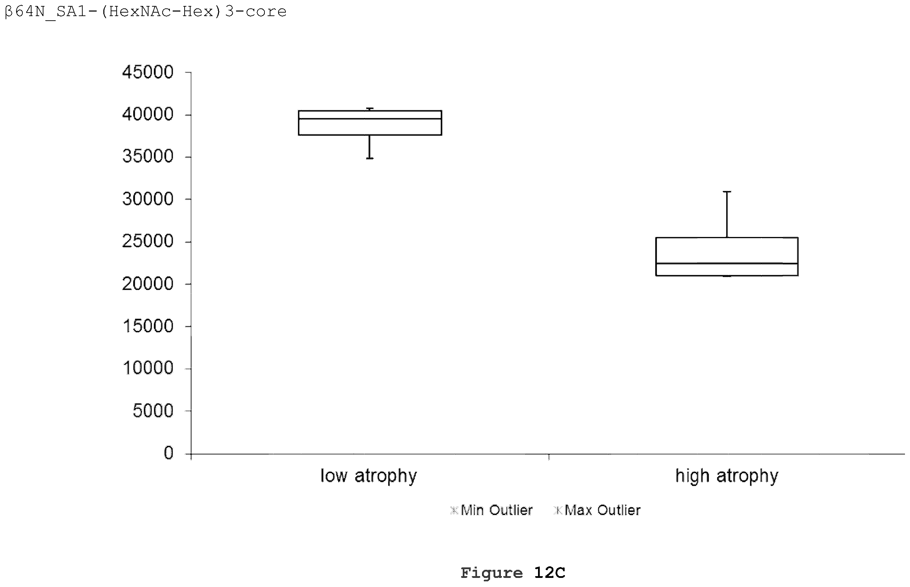

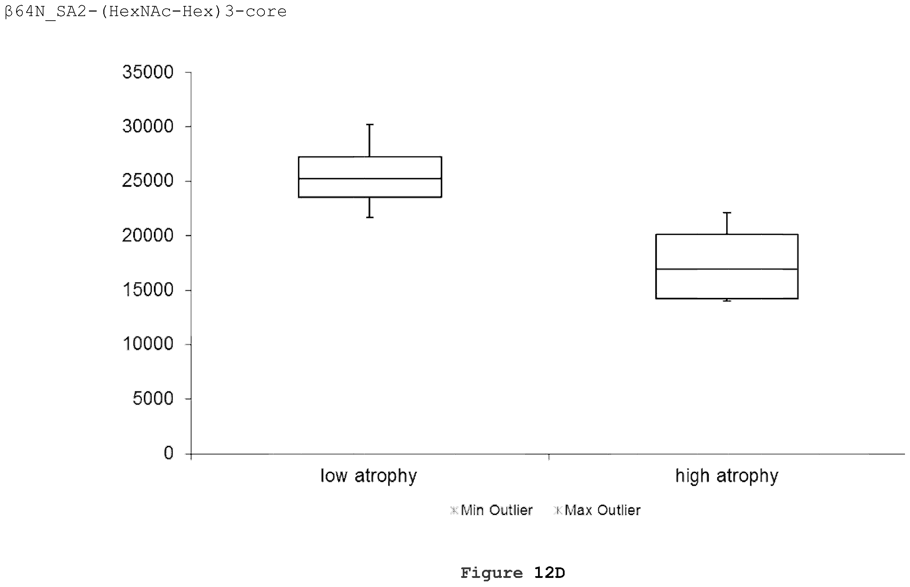

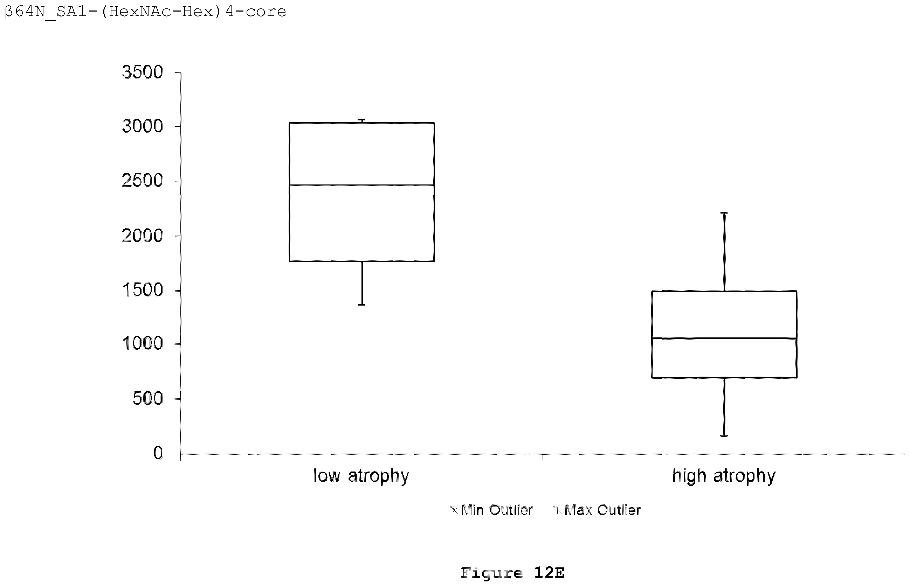

A selection of box plots for SRM quantification of individual clusterin glycoforms is provided in Figure 12.

**Table 5 Significant changes in Clusterin glycopeptides using Gel10-glyco-SRM method (4 vs 4) Discovery cohort**

| m/ z (3+) | composition | p-value* | site |
|---|---|---|---|
| 953.71 | SA1-(HexNAc-Hex)2-core | 0.0001 | β64N |
| 1050.74 | SA2-(HexNAc-Hex)2-core | 0.0004 | β64N |
| 1075.42 | SA1-(HexNAc-Hex)3-core | 0.0009 | β64N |
| 1172.45 | SA2-(HexNAc-Hex)3-core | 0.012 | β64N |
| 1197.13 | SA1-(HexNAc-Hex)4-core | 0.044 | β64N |

| | | | |
|---|---|---|---|
| *The p-value indicates significance of change between high and low atrophy groups. | | | |

### Example 7 - Validation Study of Gel10-glyco-SRM Clusterin Glycoform Selected Reaction Monitoring Assay

The eight clusterin glycopeptide Gel10-glyco-SRM assay developed in Example 6 was applied to the analysis of the Validation Cohort of Alzheimer's disease patient plasma samples comprising 9 cases with [high] level of hippocampal atrophy and 10 cases with [low] level of hippocampal atrophy. Samples were as described in Table 2 and all sample preparation and analytical methods are as described in Example 6.

Across this cohort three specific β64N site-specific glycoforms showed a statistically significantly difference between patients with high levels of hippocampal atrophy and those with lower rates of hippocampal atrophy (Table 6).

**Table 6 - Performance of Gel10-glyco-SRM Clusterin Glycoform Assay in the Validation Cohort**

| m/ z (3+) | Composition | p-value* | site |
|---|---|---|---|
| 953.71 | SA1-(HexNAc-Hex)2-core | 0.000964891 | β64N |
| 1050.74 | SA2-(HexNAc-Hex)2-core | 0.009457781 | β64N |
| 1172.45 | SA2-(HexNAc-Hex)3-core | 0.006985634 | β64N |

| | | | |
|---|---|---|---|
| *The p-value indicates significance of change between high and low atrophy groups. | | | |

When the results for the Discovery and Validation Cohorts were combined, surprisingly all eight glycoforms attained statistical significance for reduced concentrations in the high atrophy group compared to those with low hippocampal atrophy (Table 7). The power of these eight clusterin glycopeptides to differentiate patients based on their hippocampal volume provides a minimally invasive means to diagnose and predict the progression of Alzheimer's disease

**Table 7 - Combined Performance of Gel10-glyco-SRM Clusterin Glycoform SRM in combined Discovery and Validation Cohort**

| m/z (3+) | composition | p-value* | site |
|---|---|---|---|
| 953.71 | SA1-(HexNAc-Hex)2-core | 2.81311E-05 | β64N |
| 1050.74 | SA2-(HexNAc-Hex)2-core | 5.67936E-10 | β64N |
| 1075.42 | SA1-(HexNAc-Hex)3-core | 0.000662932 | β64N |
| 1172.45 | SA2-(HexNAc-Hex)3-core | 8.03747E-08 | β64N |
| 1197.13 | SA1-(HexNAc-Hex)4-core | 0.002226471 | β64N |
| 1269.49 | SA3-(HexNAc-Hex)3-core | 0.001689634 | β64N |
| 1294.17 | SA2-(HexNAc-Hex)4-core | 0.001327899 | β64N |
| 1391.53 | SA3-(HexNAc-Hex)4-core | 0.009999 | β64N |

| | | | |
|---|---|---|---|
| Box plots for each glycopeptide are provided in Figure 16 (A-H, respectively). * The p-value indicates significance of change between high and low atrophy groups. | | | |

### Conclusions

A high sensitivity SRM method for eight specific N-linked glycopeptides at β64N of human clusterin can differentiate between Alzheimer's disease cases with high hippocampal atrophy and mild cognitive impairment cases with low hippocampal atrophy. This method may provide the basis for a routine clinical test to assess hippocampal atrophy based on the detection of the level of specific glycoforms in an individual patient and comparing this to levels known to represent specific levels of hippocampal atrophy.

### References

Andreasen, N., C. Hesse, et al. (1999). "Cerebrospinal fluid beta-amyloid(1-42) in Alzheimer disease: differences between early- and late-onset Alzheimer disease and stability during the course of disease." Arch Neurol 56(6): 673-80.

Bosman, G. J., I. G. Bartholomeus, et al. (1991). "Erythrocyte membrane characteristics indicate abnormal cellular aging in patients with Alzheimer's disease." Neurobiol Aging 12(1): 13-8.

Butterfield, D. A., J. B. Owen (2011). "Lectin-affinity chromatography brain glycoproteomics and Alzheimer disease: insights into protein alterations consistent with the pathology and progression of this dementing disorder."Proteomics Clin Appl. 5(1-2):50-6

Friedland, R. P. (1993). "Epidemiology, education, and the ecology of Alzheimer's disease." Neurology 43(2): 246-9.

Ida, N., T. Hartmann, et al. (1996). "Analysis of heterogeneous A4 peptides in human cerebrospinal fluid and blood by a newly developed sensitive Western blot assay." J Biol Chem 271(37): 22908-14.

Kanai, M., E. Matsubara, et al. (1998). "Longitudinal study of cerebrospinal fluid levels of tau, A beta1-40, and A beta1-42(43) in Alzheimer's disease: a study in Japan." Ann Neurol 44(1): 17-26.

Kawarabayashi, T., L. H. Younkin, et al. (2001). "Age-dependent changes in brain, CSF, and plasma amyloid (beta) protein in the Tg2576 transgenic mouse model of Alzheimer's disease." J Neurosci 21(2): 372-81.

Killick, R., E. M. Ribe, et al. (2012). "Clusterin regulates β-amyloid toxicity via Dickkopf-1-driven induction of the wnt-PCP-JNK pathway." Mol Psychiatry. doi: 10.1038/mp.2012.163. [Epub ahead of print]

Kosaka, T., M. Imagawa, et al. (1997). "The beta APP717 Alzheimer mutation increases the percentage of plasma amyloid-beta protein ending at A beta42(43)." Neurology 48(3): 741-5.

Kuo, Y. M., T. A. Kokjohn, et al. (2000). "Elevated abeta42 in skeletal muscle of Alzheimer disease patients suggests peripheral alterations of AbetaPP metabolism." Am J Pathol 156(3): 797-805.

Lindner, M. D., D. D. Gordon, et al. (1993). "Increased levels of truncated nerve growth factor receptor in urine of mildly demented patients with Alzheimer's disease." Arch Neurol 50(10): 1054-60.

Nuutinen T., T. Suuronen, et al. (2009) "Clusterin: a forgotten player in Alzheimer's disease."Brain Res Rev. 61(2):89-104.

Pirttila, T., S. Mattinen, et al. (1992). "The decrease of CD8-positive lymphocytes in Alzheimer's disease." J Neurol Sci 107(2): 160-5.

Rocca, W. A., A. Hofman, et al. (1991). "Frequency and distribution of Alzheimer's disease in Europe: a collaborative study of 1980-1990 prevalence findings. The EURODEM-Prevalence Research Group." Ann Neurol 30(3): 381-90.

Sato, Y., T. Endo. (2010). "Alteration of brain glycoproteins during aging." Geriatr Gerontol Int 10 (Suppl. 1): 32-S40

Scheuner, D., C. Eckman, et al. (1996). "Secreted amyloid beta-protein similar to that in the senile plaques of Alzheimer's disease is increased in vivo by the presenilin 1 and 2 and APP mutations linked to familial Alzheimer's disease." Nat Med 2(8): 864-70.

Ueno, I., T. Sakai, et al. (2000). "Analysis of blood plasma proteins in patients with Alzheimer's disease by two-dimensional electrophoresis, sequence homology and immunodetection." Electrophoresis 21(9): 1832-45.

Robinson, M.D et al. (2010). "edgeR: a Bioconductor package for differential expression analysis of digital gene expression data" Bioinformatics 26: 139-140.

Paulson et al. (2013). "Differential abundance analysis for microbial marker-gene surveys" Nature Methods 10: 1200-1202.

De Livera et al. (2012). "Normalizing and Integrating Metabolomics Data" Anal. Chem. 84(24): pp. 10768-10776.

Nilselid et al. (2006). "Clusterin in cerebrospinal fluid: Analysis of carbohydrates and quantification of native and glycosylated forms" Neurochemistry International 48: 718-728.

## Claims

1. A method for assessing the hippocampal atrophy level in a test subject with Alzheimer's disease (AD), comprising:
(i) providing a protein-containing sample that has been obtained from the test subject;
(ii) determining the concentration, amount or degree of expression of at least one clusterin glycoform,
wherein tetra-antennary glycoforms of the at least one clusterin glycoform as a proportion of the total of all glycoforms of the same glycoprotein fragment are quantified;
(iii) comparing said concentration, amount or degree determined in (ii) with a reference from a control subject that does have AD with a low level of hippocampal atrophy;
(iv) based on the level of the at least one clusterin glycoform in the test subject relative to the reference, making an assessment of the hippocampal atrophy level in the test subject,
wherein a lower relative level of tetra-antennary glycoforms in the sample from the test subject compared with the relative level of tetra-antennary glycoforms in the reference from the control subject indicates that the test subject is predicted to have a relatively higher level of hippocampal atrophy,
wherein said at least one clusterin glycoform comprises a glycosylated fragment of human clusterin consisting of HN*STGCLR (SEQ ID NO: 2), wherein "N*" indicates the glycan attachment residue, and
wherein the protein-containing sample is selected from the group consisting of: blood plasma, blood cells or serum.

2. The method according to claim 1, wherein said at least one clusterin glycoform further comprises:
a) any one of the clusterin glycopeptides set forth in Table 3A, Table 3B, Table 3C, Table 5, Table 6 and/or Table 7; and/or
b) a β64N-glycan selected from the group consisting of: β64N_SA1-(HexNAc-Hex)2-core; β64N_SA2-(HexNAc-Hex)2-core; β64N_SA1-(HexNAc-Hex)3-core; β64N_SA2-(HexNAc-Hex)3-core; β64N_SA1-(HexNAc-Hex)4-core;
β64N_SA3-(HexNAc-Hex)3-core; β64N_SA2-(HexNAc-Hex)4-core; and β64N_SA3-(HexNAc-Hex)4-core.

3. The method according to claim 1 or claim 2, wherein the clusterin glycoforms are measured using:
a) gel electrophoresis; or
b) LC-MS/MS; and wherein, preferably, the relative amount of each glycoform is calculated by comparison to an equivalent heavy-isotope labelled reference glycoform using Selected Reaction Monitoring mass spectrometry.

4. The method according to any one of claims 1 to 3, wherein the clusterin glycoforms are:
a) measured using sum scaled Selected Reaction Monitoring (SRM) mass spectrometry; and/or
b) not labelled;

5. The method according to claim 3, wherein the heavy-isotope labelled reference clusterin glycoform is
a) a synthetic glycopeptide wherein one or more heavy isotopes of H, C, N or O are substituted within the peptide or sugar components of said glycoform; or
b) an enriched, naturally occurring clusterin glycoform that has been labelled with an isotopic mass tag wherein said isotopic mass tag comprises one or more heavy isotopes of H, C, N or O and wherein such mass tag is able to react with the peptide or sugar components of said glycoform.

6. The method according to claim 3, wherein the relative amount of each clusterin glycoform is calculated by comparison to an equivalent clusterin glycoform labelled with an isobaric mass tag wherein:
(i) each sample taken from the test subject is labelled with one member of an isobaric mass tag set to create a labelled analytical sample;
(ii) a standard reference panel of enriched clusterin glycoforms is separated into between two and six aliquots and each aliquot is labelled separately with additional members of the same isobaric mass tag set as the labelled analytical sample and each independently labelled aliquot of the reference panel is mixed in a predefined ratio to create a clinically relevant concentration curve as a standard reference mixture;
(iii) an equal volume of the labelled analytical sample and the standard reference mixture are mixed together to form the MS calibrator sample; and
(iv) the MS calibrator sample prepared in step (iii) is analysed by mass spectrometry;
wherein, preferably, the isobaric mass tag set is a Tandem Mass Tag set.

7. The method according to any one of the preceding claims, wherein the at least one clusterin glycoform is measured by an immunological assay, wherein, preferably the immunological assay comprises Western blotting or ELISA.

8. A method for stratifying a plurality of test subjects according to their stage of Alzheimer's disease (AD), comprising:
carrying out the method according to any one of claims 1 to 7 on at least one test sample from each of the human test subjects; and
based on the level of the at least one specific clusterin glycoform in each of the test subjects, stratifying the test subjects into more or less advanced stage AD; wherein the test subjects are stratified according to their predicted degree of hippocampal atrophy.

9. A method of determining the efficacy of a treatment of Alzheimer's disease (AD)in a mammalian subject, comprising:
determining the level of one or more clusterin glycoforms by carrying out the method according to any one of claims 1 to 7 before treatment of the subject and at least one time during or following treatment of the subject,
wherein successful treatment is demonstrated by the level of the said one or more clusterin glycoforms remaining stable or reverting to more normal levels, wherein, the subject is human.

## Patentansprüche

1. Verfahren zur Bewertung des Grades von hippocampaler Atrophie bei einer Versuchsperson/einem Versuchstier mit Alzheimer-Krankheit (AD), mit den Schritten:
(i) Bereitstellen einer proteinhaltigen Probe, die aus der Versuchsperson/dem Versuchstier gewonnen worden ist;
(ii) Bestimmen der Konzentration, Menge oder des Ausmaßes von Expression von mindestens einer Clusterin-Glykoform,
wobei Tetra-Antennen-Glykoformen der mindestens einen Clusterin-Glykoform als ein Anteil der Gesamtheit aller Glykoformen ein und desselben Glykoprotein-Fragments quantifiziert werden;
(iii) Vergleichen der in (ii) bestimmten Konzentration, Menge oder des Ausmaßes mit einem Bezugswert aus einer Kontrollperson, die AD mit einem geringen Grad an hippocampaler Atrophie hat;
(iv) Abgeben einer Bewertung des hippocampalen Atrophiegrades bei der Versuchsperson/dem Versuchstier auf der Basis der Höhe der mindestens einen Clusterin-Glykoform bei der Versuchsperson/dem Versuchstier bezüglich des Bezugswertes,
wobei ein geringerer relativer Spiegel von Tetra-Antennen-Glykoformen in der Probe aus der Versuchsperson/dem Versuchstier im Vergleich zu dem relativen Spiegel von Tetra-Antennen-Glykoformen in dem Bezugswert aus der Kontrollperson anzeigt, dass der Versuchsperson/dem Versuchstier ein relativ höherer Grad an hippocampaler Atrophie prognostiziert wird,
wobei die mindestens eine Clusterin-Glykoform ein glykosyliertes Fragment von menschlichem Clusterin bestehend aus HN*STGCLR (SEQ ID NR: 2) aufweist, wobei "N*" den Glykan-Adhäsionsrest anzeigt, und
wobei die proteinhaltige Probe aus der Gruppe bestehend aus: Blutplasma, Blutzellen oder -serum ausgewählt wird .

2. Verfahren nach Anspruch 1, wobei die mindestens eine Clusterin-Glykoform weiterhin Folgendes aufweist:
a) eines der in Tabelle 3A, Tabelle 3B, Tabelle 3C, Tabelle 5, Tabelle 6 und/oder Tabelle 7 dargelegten Clusterin-Glykopeptide; und/oder
b) ein β64N-Glykan ausgewählt aus der Gruppe bestehend aus: β64N_SA1- (HexNAc-Hex) 2-Kern; β64N_SA2- (HexNAc-Hex) 2-Kern; β64N_SA1- (HexNAc-Hex) 3-Kern; β64N_SA2- (HexNAc-Hex) 3-Kern; β64N_SA1- (HexNAc-Hex) 4-Kern; β64N_SA3- (HexNAc-Hex) 3-Kern; β64N_SA2- (HexNAc-Hex) 4-Kern; sowie β64N_SA3- (HexNAc-Hex) 4-Kern.

3. Verfahren nach Anspruch 1 oder Anspruch 2, wobei die Clusterin-Glykoformen gemessen werden unter Einsatz von:
a) Gel-Elektrophorese; oder
b) LC-MS/MS; und wobei vorzugsweise die relative Menge jeder Glykoform durch Vergleich mit einer als Bezugs-Glykoform gekennzeichneten äquivalenten Schwerisotop unter Einsatz von Selected Reaction Monitoring (Ausgewählte Reaktionsüberwachung) Massenspektrometrie berechnet wird.

4. Verfahren nach einem der Ansprüche 1 bis 3,
wobei die Clusterin-Glykoformen:
a) unter Einsatz von summenskalierter Selected Reaction Monitoring (SRM) Massenspektrometrie gemessen werden; und/oder
b) nicht markiert sind.

5. Verfahren nach Anspruch 3, wobei das als Bezugs-Clusterin-Glykoform markierte Schwerisotop
a) ein synthetisches Glykopeptid ist, wobei ein oder mehr Schwerisotope von H, C, N oder O in dem Peptid oder Zuckerbestandteilen der Glykoform ersetzt sind; oder
b) eine angereicherte, natürlich vorkommende Clusterin-Glykoform ist, das mit einer Isotopenmassenmarkierung markiert worden ist, wobei die Isotopenmassenmarkierung ein oder mehr Schwerisotope von H, C, N oder O aufweist, und wobei die Massenmarkierung mit dem Peptid oder Zuckerbestandteilen der Glykoform reagieren kann.

6. Verfahren nach Anspruch 3, wobei die relative Menge jeder Clusterin-Glykoform im Vergleich zu einer mit einer isobaren Massenmarkierung markierten, äquivalenten Clusterin-Glykoform berechnet wird, wobei:
(i) jede aus der Versuchsperson/dem Versuchstier entnommene Probe mit einem Element eines isobaren Massenmarkierungssatzes markiert ist, um eine markierte analytische Probe zu erzeugen;
(ii) eine Standardbezugsreihe aus angereicherten Clusterin-Glykoformen in zwischen zwei und sechs Aliquoten zerlegt wird und jede Aliquote getrennt mit zusätzlichen Elementen desselben isobaren Massenmarkierungssatzes als die markierte analytische Probe markiert wird und jede unabhängig markierte Aliquote der Bezugsreihe in einem vorgegebenen Verhältnis gemischt wird, um eine klinisch relevante Konzentrationskurve als eine Standardbezugsmischung zu erzeugen;
(iii) ein gleiches Volumen der markierten analytischen Probe und der Standardbezugsmischung zusammengemischt werden, um die MS-Eichprobe herzustellen; sowie
(iv) die in Schritt (iii) hergestellte MS Eichprobe durch Massenspektroskopie analysiert wird;
wobei der isobare Massenmarkierungssatz vorzugsweise ein Tandem-Massenmarkierungssatz ist.

7. Verfahren nach einem der vorhergehenden Ansprüche, wobei das mindestens eine Clusterin-Glykoform durch ein immunologisches Nachweisverfahren gemessen wird, wobei das immunologische Nachweisverfahren vorzugsweise Western-Blotting oder ELISA umfasst.

8. Verfahren zur Schichtung einer Mehrzahl von Versuchspersonen/Versuchstieren gemäß ihrem Stadium von Alzheimer-Krankheit (AD), mit den Schritten:
Ausführen des Verfahrens nach einem der Ansprüche 1 bis 7 an mindestens einer Probe aus jedem der menschlichen Versuchsobjekte; und
auf der Basis des Spiegels der mindestens einen speziellen Clusterin-Glykoform in jedem der Versuchsobjekte, Schichten der Versuchspersonen/Versuchstiere in mehr oder weniger fortgeschrittenes AD-Stadium; wobei die Versuchspersonen/Versuchstiere gemäß ihrem prognostizierten Grad von hippocampaler Atrophie geschichtet werden.

9. Verfahren zur Bestimmung der Wirksamkeit einer Behandlung von Alzheimer-Krankheit (AD) in einem Versuchssäugetier, mit den Schritten:
Bestimmen des Spiegels von einer oder mehr Clusterin-Glykoformen durch Ausführung des Verfahrens nach einem der Ansprüche 1 bis 7 vor der Behandlung des Objekts und mindestens einmal während oder nach der Behandlung des Objekts,
wobei eine erfolgreiche Behandlung durch den Spiegel der einen oder mehreren Clusterin-Glykoformen aufgezeigt wird, die stabil bleiben oder zu normalen Spiegeln zurückkehren, wobei das Objekt ein Mensch ist.

## Revendications

1. Une méthode d'évaluation du niveau d'atrophie de l'hippocampe chez un sujet test atteint de la maladie d'Alzheimer (MA) comprenant :
i) la fourniture d'un échantillon contenant une protéine prélevée sur le sujet test;
ii) la détermination de la concentration, en quantité ou en taux d'expression d'au moins une glycoforme de la clustérine,
dans laquelle sont quantifiées les glycoformes tétra-antennes de la au moins une glycoforme de la clustérine en tant que proportion du total de toutes les glycoformes du même fragment de glycoprotéine ;
iii) la comparaison de ladite concentration, en quantité ou en taux d'expression déterminé en ii) avec une référence provenant d'un sujet témoin atteint d'Alzheimer présentant un faible niveau d'atrophie de l'hippocampe ;
(iv) sur la base du niveau de la au moins une glycoforme de la clustérine chez le sujet test par rapport au sujet de référence, procéder à une évaluation du niveau d'atrophie de l'hippocampe chez le sujet test,
dans laquelle un niveau relatif inférieur de glycoformes tétra-antennes dans l'échantillon prélevé sur le sujet test comparé avec le niveau relatif de glycoformes tétra-antennes dans le résultat provenant d'un sujet témoin prévoit que le sujet test devrait atteindre un niveau d'atrophie de l'hippocampe relativement plus élevé,
dans laquelle ladite au moins une glycoforme de la clustérine comprend un fragment glycosylé de clustérine humaine consistant en HN^{∗}STGCLR (SEQ ID NO: 2), où «N^{∗}» indique le résidu de fixation de glycane, et
dans laquelle l'échantillon contenant la protéine est choisi dans le groupe consistant en plasma sanguin, en cellules sanguines ou en sérum.

2. La méthode selon la revendication 1, dans laquelle ladite au moins une glycoforme de la clustérine comprend en outre :
a) l'un quelconque des glycopeptides de clustérine figurant dans le Tableau 3A, le Tableau 3B, le Tableau 3C, le Tableau 5, le Tableau 6 et/ou le Tableau 7 ; et/ou
b) un β64N-glycan choisi dans le groupe composé de:
β64N_SAI-(HexNAc-Hex) 2-noyau ; β64N-SA2-(HexNAcHex) 2-noyau ;
β64N_SAI-(HexNAc-Hex) 3-noyau ; β64N_SA2-(HexNAc-Hex) 3-noyau ;
β64N_SAI-(HexNAc-Hex) 4-noyau ; β64N_SA3-(HexNAc-Hex) 3-noyau ;
β64N_SA2-(HexNAc-Hex) 4-noyau ; et β64N_SA3-(HexNAc-Hex) 4-noyau.

3. La méthode selon la revendication 1 ou la revendication 2, dans laquelle les glycoformes de la clustérine sont mesurées en utilisant :
a) l'électrophorèse sur gel ; ou
b) LC-MS/MS ; et dans lequel, de préférence, la quantité relative de chaque glycoforme est calculée par comparaison avec une glycoforme de référence marquée par des isotopes lourds équivalente en utilisant la spectrométrie de masse avec Surveillance des Réactions Sélectionnées.

4. La méthode selon l'une quelconque des revendications 1 à 3, dans laquelle les glycoformes de la clustérine sont
a) mesurées en utilisant la spectrométrie de masse avec Surveillance des Réactions Sélectionnées (SRM) mise à l'échelle de la somme, et/ou
b) non marquées.

5. La méthode selon la revendication 3, dans laquelle la glycoforme de la clustérine de référence marquée aux isotopes lourds est :
a) un glycopeptide synthétique dans lequel un ou plusieurs isotopes lourds sont substitués à H, C, N ou O dans les composants peptidiques ou composants sucrés de ladite glycoforme ; ou
b) une glycoforme de la clustérine enrichie et existant naturellement qui a été marquée d'une étiquette de masse isotopique dans laquelle ladite étiquette de masse isotopique comprend un ou plusieurs isotopes lourds de H, C, N ou O et dans laquelle cette étiquette de masse est capable de réagir avec les composants peptidiques ou sucrés de ladite glycoforme.

6. La méthode selon la revendication 3, dans laquelle la quantité relative de chaque glycoforme de la clustérine est calculée par comparaison avec une glycoforme de la clustérine marquée équivalente comportant une étiquette de masse isobare, où :
i) chaque échantillon prélevé sur le sujet test est marqué d'un élément d'un ensemble d'étiquettes de masse isobare pour créer un échantillon analytique marqué ;
ii) un panel de référence standard de glycoformes de la clustérine enrichies est séparé en deux à six parties aliquotes et chaque partie aliquote est marquée séparément de membres supplémentaires du même ensemble d'étiquettes de masse isobare que l'échantillon analytique marqué et chaque partie aliquote marquée indépendamment du panel de référence est mélangée selon un rapport prédéfini pour créer une courbe de concentration cliniquement pertinente en tant que mélange de référence standard ;
iii) un volume égal de l'échantillon analytique marqué et du mélange de référence standard est mélangé pour former l'échantillon d'étalonnage MS ; et
iv) l'échantillon d'étalonnage MS obtenu dans l'étape iii) est analysé par spectrométrie de masse ;
dans lequel, de préférence, l'ensemble d'étiquettes de masse isobare est un ensemble d'étiquettes de masse tandem.

7. La méthode selon l'une quelconque des revendications précédentes, dans laquelle ladite au moins une glycoforme de la clustérine est mesurée par dosage immunologique, dans lequel, de préférence, le dosage immunologique comprend la technique des immuno- empreintes (Western blotting) ou ELISA.

8. Une méthode de stratification d'une pluralité de sujets tests en fonction de leur stade de la maladie d'Alzheimer (MA) comprenant :
la mise en œuvre de la méthode selon l'une quelconque des revendications 1 à 7 sur au moins un échantillon test prélevé sur chacun des sujets test humains ; et
en fonction du niveau de ladite au moins une glycoforme de la clustérine spécifique chez chacun des sujets de test, l'étape consistant à stratifier les sujets de test en stades AD plus ou moins avancés ; les sujets de test étant stratifiés en fonction de leur degré prédit d'atrophie de l'hippocampe.

9. Une méthode de détermination de l'efficacité d'un traitement de la maladie d'Alzheimer (MA) chez un sujet mammifère, comprenant :
la détermination du niveau d'une ou plusieurs glycoformes de la clustérine, par mise en œuvre de la méthode selon l'une quelconque des revendications 1 à 7 avant le traitement du sujet et au moins une fois, pendant ou après le traitement du sujet, dans lequel le succès du traitement est démontré par le niveau de ladite une ou plusieurs glycoformes de la clustérine restant stables ou revenant à des niveaux plus normaux, le sujet étant humain.
